Europäisches Patentamt

European Patent Office

Office européen des brevets

(19)

(11) Veröffentlichungsnummer : **0 362 222 B1**

(12) **EUROPÄISCHE PATENTSCHRIFT**

(45) Veröffentlichungstag der Patentschrift :
**12.08.92 Patentblatt 92/33**

(51) Int. Cl.⁵ : **A61M 5/20**

(21) Anmeldenummer : **88903833.7**

(22) Anmeldetag : **05.05.88**

(86) Internationale Anmeldenummer :
**PCT/EP88/00375**

(87) Internationale Veröffentlichungsnummer :
**WO 88/08725 17.11.88 Gazette 88/25**

(54) **INJEKTIONSGERÄT MIT EINEM SPANNGLIED UND MIT EINEM ZWEITEN STELLGLIED.**

(30) Priorität : **08.05.87 DE 3715258**

(43) Veröffentlichungstag der Anmeldung :
**11.04.90 Patentblatt 90/15**

(45) Bekanntmachung des Hinweises auf die
Patenterteilung :
**12.08.92 Patentblatt 92/33**

(84) Benannte Vertragsstaaten :
**AT BE CH DE FR GB IT LI NL SE**

(56) Entgegenhaltungen :
**EP-A- 0 058 536**
**WO-A-87/02895**
**DE-A- 3 527 066**
**US-A- 2 565 081**
**US-A- 3 815 785**

(73) Patentinhaber : **WILHELM HASELMEIER
GMBH & CO.
Vaihinger Strasse 48 Postfach 81 02 60
W-7000 Stuttgart 80 (DE)**

(72) Erfinder : **BECHTOLD, Herbert
Breslauer Str. 19
W-7031 Ehningen (DE)**
Erfinder : **GABRIEL, Jochen
Im Falkenrain 1
W-7000 Stuttgart 1 (DE)**

(74) Vertreter : **Raible, Hans, Dipl.-Ing.
Schoderstrasse 10
W-7000 Stuttgart 1 (DE)**

## Beschreibung

Die Erfindung betrifft ein Injektionsgerät mit einem zum Bewirken eines Injektionsvorgangs dienenden Organ, welch letzteres mittels eines Spannglieds entgegen der Kraft einer Feder in eine Spannstellung bringbar ist, aus der es zum Bewirken eines Injektionsvorgangs freigebbar ist, um eine Wand, insbesondere einen Kolben, eines eine zu injizierende Flüssigkeit enthaltenden Behälters zu beaufschlagen.

Aus der US-A- 4 194 505 ist ein Injektionsgerät bekannt, das hauptsächlich zur Injektion von Insulin dient. Dieses Gerät hat ein zum Bewirken eines Injektionsvorganges dienendes Organ, das entgegen der Kraft einer Feder in eine Spannstellung bringbar ist, aus der es freigebbar ist, um den Kolben einer Kartusche zu beaufschlagen, die Insulin enthält. Am Gehäuse dieses bekannten Injektionsgeräts ist ein Verstellglied vorgesehen, das als Anschlag für das genannte Organ dient und es ermöglicht, die wirksame Länge dieses Organs und damit die Injektionsdosis innerhalb bestimmter Grenzen zu verändern. Am Gehäuse ist eine Skala vorgesehen, mit der das Verstellglied auf einen gewünschten Wert eingestellt werden kann. Das bekannte Gerät ist ausgebildet zur Aufnahme spezieller Kartuschen, die eine bestimmte Menge Insulin enthalten, beispielsweise 20 Einheiten. Benötigt der Patient nur 17 Einheiten, so hat er die Möglichkeit, am Verstellglied eine entsprechende Einstellung vorzunehmen. Dazu stellt er zunächst die Maximaldosis ein, welche einer Nullinie auf der Skala des Geräts entspricht. Ausgehend hiervor stellt er mit dem Verstellglied auf dieser Skala drei Striche ein, entsprechend den drei Einheiten, die er nicht injizieren möchte. Dadurch wird am Gehäuse der Anschlag für das bewegliche Organ verstellt. Anschließend löst der Patient die Injektion aus, wobei zunächst die Injektionsnadel eingestochen und dann die gewünschte Insulindosis aus der Kartusche injiziert wird. - Dieses Gerät ist kompliziert zu bedienen: Man benötigt hierfür Kartuschen mit unterschiedlicher Dosierung, nämlich solche mit 10, 20, 30, 40, 50 und 60 Einheiten Insulin. Ist der Patient blind, so kann er solche Kartuschen leicht verwechseln, und es besteht dann die Gefahr, daß er sich zu viel Insulin injiziert. Auch sind die Rechenoperationen bei der Bedienung dieses Geräts nicht gerade einfach und können für manche Patienten zu schwierig sein. Bei der sogenannten intensivierten Insulintherapie muß nämlich der Patient nach jeder Mahlzeit eine andere Quantität Insulin injizieren, und es kann dann leicht zu Rechenfehlern kommen, oder der Patient verwendet einfach die alte Einstellung des Geräts weiter. Benötigt der Patient z.B. nach dem Frühstück 8 Einheiten, nach dem Mittagessen 15 Einheiten und nach dem Abendessen 12 Einheiten Insulin, so muß er bei diesem bekannten Gerät zum Frühstück eine Kartusche für 10 Einheiten verwenden und das Gerät auf minus zwei Einheiten einstellen. Zum Mittag- und Abendessen muß er eine Kartusche mit 20 Einheiten verwenden und dabei das Gerät mittags auf minus fünf Einheiten und abends auf minus acht Einheiten einstellen. All das erfordert viel Sorgfalt und kann leicht zu Fehlern führen.

Ferner kennt man aus der DE-A1- 3 527 066 einen sogenannten Panseninjektor. Bei diesem Gerät hat die Kolbenstange des Injektionskolbens an ihrem freien Ende einen Handgriff, und diesem Handgriff gegenüberliegend ist am Gerät ein verstellbarer Anschlag vorgesehen, der die Bewegungen dieses Handgriffs begrenzt und dadurch die Injektionsdosis bestimmt. Bei diesem Gerät sticht der Tierarzt die Kanüle von Hand in das betreffende Tier ein, und durch das Einstechen wird ein automatischer Injektionsvorgang ausgelöst, bei dem die Kolbenstange durch eine Feder so weit in den Injektionszylinder gepreßt wird, bis der Handgriff gegen den erwähnten Anschlag stößt. - Nachteilig bei diesem Gerät ist, daß man den Injektionszylinder vor jeder Injektion neu laden muß. Dies erlaubt nicht die Verwendung von Kartuschen oder sonstigen Behältern, die mit mehreren Injektionsdosen der zu injizierenden Flüssigkeit gefüllt sind.

Aus der EP-A1- 0 058 536 kennt man ein Injektionsgerät, mit dem ein Patient aus einer Kartusche mit Insulin mehrere Dosen injizieren kann. Er stellt dazu durch Verdrehen eines Verstellrades die gewünschte Insulindosis ein, sticht dann die Nadel ein, und dreht anschließend das Voreinstellrad in dessen Nullstellung zurück. Dadurch wird ein Gewindeteil aus einer Hülse herausgeschraubt, drückt gegen den Kolben in der Kartusche, und preßt Insulin aus der Kartusche durch die Injektionsnadel in den Patienten. Nachteilig hierbei ist aber, daß dieses Gerät nur auf der Vorderseite des Körpers verwendet werden kann, während man bei der sogenannten intensivierten Insulintherapie in der Praxis gezwungen ist, auch Injektionen auf der Rückseite des Körpers vorzunehmen, um Hämatome und Hornhautbildung durch zu häufiges Injizieren an derselben Stelle zu vermeiden. Auch ist eine Injektion durch manuelles Drehen eines Voreinstellrades für manche Patienten sehr schmerzhaft.

Injektionsgeräte der geschilderten Art sind komplizierte Geräte. Deshalb muß der Patient, gewöhnlich in einem Krankenhaus, sorgfältig in ihrer Bedienung unterwiesen werden. Zusätzlich ist es wünschenswert, die Bedienung derartiger Geräte so einfach und verständlich wie möglich zu machen, damit dem Patienten die Benutzung nicht lästig wird und er seine Therapie regelmäßig fortsetzt und dabei keine Fehler macht.

Nach der Erfindung wird diese Aufgabe gelöst durch eine Steueranordnung, welche verschiedene Informationen des Geräts auswertet. Durch die relative Stellung eines ersten und eines zweiten Stellglieds erhält die Steueranordnung Informationen, und diese werden in der Steueranordnung ausgewertet, um die Benutzung sicherer und - soweit das innerhalb der Größe eines solchen kleinen Geräts realisierbar ist - fehlerfrei

EP 0 362 222 B1

zu machen. Man erhält so sozusagen einen internen Computer in Form dieser Steueranordnung, und dieser Computer wertet die Informationen aus, die ihm durch die Stellungen von erstem und zweitem Stellglied zugeführt werden und verarbeitet diese Informationen zu entsprechenden "Befehlen", die Fehlbedienungen durch den Patienten verhindern sollen. Die Steueranordnung hat ein Eingriffsglied. Nach dem Spannen eines Spannglieds steht dieses Eingriffsglied mit einem ersten Abschnitt eines Gegenglieds, nach dem Injektionsvorgang mit einem zweiten Abschnitt dieses Gegenglieds in Eingriff. Beim Gegenstand des Anspruchs 1 hat der zweite Abschnitt des Gegenglieds einen Anschlag für den Eingriff mit dem Eingriffsglied und begrenzt so die relative Verdrehung zwischen dem ersten Stellglied, das gleichzeitig als Spannglied dient, und dem zweiten Stellglied, so daß der Patient das zvreite Stellglied nach der Injektion leicht bis zu diesem Anschlag nachstellen kann, auch wenn er z.B. schlecht sieht.

Beim Gegenstand des Anspruchs 2 hat der zvreite Abschnitt des Gegenglieds eine Steuerausnehmung für den Eingriff des Eingriffsglieds Diese Steuerausnehmung verhindert nach der Injektion ein erneutes Spannen des Spanngliedes, wenn der Patient nicht zuvor eine bestimmte relative Drehstellung zwischen den beiden Stellgliedern eingestellt hat. Dies erinnert den Patienten daran, daß er vor dem Spannen an seinem Gerät eine bestimmte Einstellung vornehmen muß, z.B., daß er gemäß Anspruch 3 die Skalenanzeige seines Geräts auf Null stellen muß, da er sonst das Gerät gar nicht spannen kann. Dies erschwert oder verhindert Fehlbedienungen. Eine besonders vorteilhafte Ausgestaltung des zweiten Abschnitts des Gegenglieds ist Gegenstand des Anspruchs 4.

Beim Gegenstand des Anspruchs 5 hat der erste Abschnitt des Gegenglieds Ratschenzähne zum Eingriff mit dem Eingriffsglied. Dies ermöglicht eine Dosiseinstellung durch den Benutzer erst dann, wenn das Gerät bereits gespannt ist, wobei die Einstellung auch für einen sehschwachen oder blinden Patienten möglich ist. Die Dosiseinstellung erst nach dem Spannen hat den Vorteil, daß der Patient nicht von der vorherigen Injektion eine Dosiseinstellung übernehmen kann; dies ist gerade bei der intensivierten Insulintherapie wichtig, da hierbei je nach Tageszeit sehr unterschiedliche Insulinmengen injiziert werden müssen. Dabei werden die Ratschenzähne bevorzugt nach Anspruch 6 ausgebildet, um beim Injektionsvorgang ein axiales Herausgleiten des Eingriffsgliedes aus den Ratschenzähnen zu ermöglichen.

Beim Gegenstand des Anspruchs 7 hat der erste Abschnitt des Gegenglieds eine Sperrvorrichtung, welche bei einer vorgegebenen relativen Drehstellung zwischen Eingriffsglied und Gegenglied einen Injektionsvorgang blockiert. Dadurch wird verhindert, daß der Patient sofort nach dem Spannen des Geräts eine Injektion auslöst, ohne zuvor eine Injektionsdosis eingestellt zu haben. Versucht der Patient, direkt nach dem Spannen eine Injektion auszulösen, so wird er daran erinnert, daß er nach dem Spannen zuerst die richtige Injektionsdosis einstellen muß.

Beim Gegenstand des Anspruchs 8 hat der erste Abschnitt des Gegenglieds einen Anschlag für den Eingriff mit dem Eingriffsglied Dies begrenzt die Einstellung der Injektionsdosis nach oben und verhindert, daß z.B. ein verwirrter Patient eine weit überhöhte Injektionsdosis einstellt.

Beim Gegenstand des Anspruchs 9 sind Steuerausnehmungen und Eingriffsglied so ausgebildet, daß sie beim Einstellen - im gespannten Zustand des Injektionsgeräts - nur eine Erhöhung der Injektionsdosis ermöglichen. Dies verhindert, daß z. B. ein sehschwacher Patient seine Dosis zunächst erhöht, dann reduziert, und schließlich nicht mehr weiß, was er überhaupt eingestellt hat.

Sehr vorteilhaft ist die Ausgestaltung nach Anspruch 10, da sie in sehr einfacher Weise eine Anzeige der eingestellten Injektionsdosis ermöglicht. Die Skala kann nach der Injektion wieder auf Null zurückgestellt werden. Das erspart dem Patienten komplizierte und fehlerbehaftete Rechenoperationen.

Gemäß Anspruch 11 wird das zweite Stellglied in sehr bevorzugter und vorteilhafter Weise über eine Rutschkupplung mit dem Gehäuse verbunden. Dies ermöglicht eine stufenlose Nachstellung des zweiten Stellglieds. Naturgemäß könnte man statt einer Rutschkupplung im Rahmen der Erfindung auch z.B. ein Ratschengesperre verwenden, wobei dann eine Nachstellung des zweiten Stellglieds nur in diskreten Schritten möglich wäre.

In besonders vorteilhafter Weise wird gemäß Anspruch 12 diese Rutschkupplung (oder das Ratschengesperre etc. ) so ausgebildet, daß das zweite Stellglied - das ja im Betrieb auch einen Anschlag für die Drehbewegungen des Spannglieds bilden muß - erst nach

Überwinden eines nicht unbeträchtlichen Losbrechmoments verdreht werden kann. Das zweite Stellglied erfüllt so ebenfalls eine Mehrfachfunktion, und zwar in einer sehr einfachen, raumsparenden Weise.

Dabei ist es besonders vorteilhaft, wenn die Skalenanzeige sowohl im gespannten wie im ungespannten Zustand sichtbar ist, denn hierdurch bleibt der Wert der Injektionsdosis zunächst "gespeichert", und der Patient kann z.B. beim Mittagessen nochmals nachsehen, wieviel Insulin er sich am Morgen injiziert hat. Diese scheinbar einfache Maßnahme stellt einen wichtigen Sicherheitsfortschritt dar.

Mit besonderem Vorteil geht man gemäß Anspruch 13 vor, da dies ein - regelmäßig sehr schmerzhaftes - Einklemmen der Finger beim Injektionsvorgang weitgehend verhindert.

EP 0 362 222 B1

Anspruch 16 betrifft die Verbindung der Steueranordnung mit dem in seiner Länge verstellbaren Organ, und Anspruch 17 betrifft die bevorzugte Ausbildung dieses in seiner Länge verstellbaren Organs.

Eine besonders einfache und kompakte Ausgestaltung ergibt sich gemäß Anspruch 18, da das Führungsglied hierbei mehrere Funktionen gleichzeitig übernimmt und sehr leicht montiert werden kann. Eine besonders vorteilhafte Ausgestaltung des Führungsglieds ist dann im Anspruch 19 angegeben. Eine sehr vorteilhafte und einfache Auslösevorrichtung ist Gegenstand des Anspruchs 20.

Der Gegenstand des Anspruchs 21 ermöglicht eine sogenannte vollautomatische Injektion, d.h. der Patient setzt nur noch das gespannte und richtig eingestellte Injektionsgerät an der gewünschten Körperstelle an, betätigt eine Auslösung, und sowohl das Einstechen der Kanüle wie die Injektion der eingestellten Dosis laufen automatisch ab. Dies ermöglicht die Injektion auch am Rücken und ist deshalb besonders wichtig für die sogenannte intensivierte Insulintherapie.

Weitere Einzelheiten und vorteilhafte Weiterbildungen der Erfindung ergeben sich aus dem im folgenden beschriebenen und in der Zeichnung dargestellten, in keiner Weise als Einschränkung der Erfindung zu verstehenden Ausführungsbeispiel, sowie aus den Unteransprüchen. Es zeigt:

Fig. 1 eine Seitenansicht eines erfindungsgemäßen Injektionsgeräts, in vergrößertem Maßstab und in der Stellung nach Vornahme einer Injektion, in Draufsicht,

Fig. 2 eine Darstellung analog Fig. 1, aber relativ zu dieser um 90° verdreht, im Längsschnitt,

Fig. 3 eine Einzelheit der Fig. 2, in stark vergrößertem Maßstab,

Fig. 4 einen Längsschnitt durch das Injektionsgerät, analog Fig. 2, aber im gespannten Zustand, also vor der Injektion,

Fig. 5 eine Einzelheit der Fig. 4, in stark vergrößertem Maßstab,

Fig. 6 eine auseinandergezogene, raumbildliche Darstellung des sogenannten B-Teils des Injektionsgeräts, welches zur Aufnahme der Kartusche mit dem zu injizierenden Stoff dient,

Fig. 7 eine raumbildliche Darstellung des Steuerteils für die Steuerung der Injektionsmenge,

Fig. 8 eine raumbildliche Darstellung des hinsichtlich seiner Länge verstellbaren Stößels, in einem gegenüber Fig. 7 verkleinerten Maßstab,

Fig. 9 einen Längsschnitt durch eine zur Einstellung der Injektionsmenge dienende Einstellhülse, gesehen längs der Linie IX-IX der Fig. 13,

Fig. 10 eine Draufsicht auf die Einstellhülse der Fig. 9,

Fig. 11 einen Längsschnitt durch die Einstellhülse der Fig. 9 und 10, gesehen längs der Linie XI-XI der Fig. 13,

Fig. 12 einen Schnitt, gesehen längs der Linie XII-XII der Fig. 10,

Fig. 13 einen Schnitt, gesehen längs der Linie XIII-XIII der Fig. 10,

Fig. 14 eine Seitenansicht der Einstellhülse, gesehen längs der Linie XIV-XIV der Fig. 10,

Fig. 14A eine Seitenansicht der Einstellhülse, gesehen längs der Linie XIVA-XIVA der Fig. 10,

Fig. 15 ein schematisches Schaubild in Form einer Abwicklung der Einstellhülse, zur Erläuterung der Erfindung,

Fig. 16 eine raumbildliche Darstellung einer bei der Einspritzmengeneinstellung verwendeten Sperrklinke, in stark vergrößertem Maßstab,

Fig. 17 einen Längsschnitt durch die Sperrklinke der Fig. 16, gesehen längs der Linie XVII-XVII der Fig. 19,

Fig. 18 eine seitliche Draufsicht auf die Sperrklinke der Fig. 16 und 17,

Fig. 19 eine Ansicht, gesehen längs der Linie XIX-XIX der Fig. 17,

Fig. 20 einen Schnitt, gesehen längs der Linie XX-XX der Fig. 17,

Fig. 21 eine Seitenansicht eines zur Längsführung des Stößels dienenden Kunststoffteils, gesehen längs der Linie XXI-XXI der Fig. 22,

Fig. 22 einen Längsschnitt, gesehen längs der Linie XXII-XXII der Fig. 21,

Fig. 23 eine Seitenansicht, gesehen längs der Linie XXIII-XXIII der Fig. 22,

Fig. 24 eine Draufsicht, gesehen längs des Pfeiles XXIV der Fig. 22,

Fig. 25 eine Draufsicht auf das hintere (distale) Gehäuseteil, welche das in diesem vorgesehene Fenster zeigt, das bei der Auslösung des Injektionsvorgangs Verwendung findet,

Fig. 26 einen Längsschnitt, gesehen längs der Linie XXVI-XXVI der Fig. 25,

Fig. 27 einen Längsschnitt durch ein hülsenartiges Teil, welches in das hintere Gehäuseteil der Fig. 25 und 26 eingesetzt wird, gesehen längs der Linie XXVII-XXVII der Fig. 28, und

Fig. 28 eine Draufsicht auf das hülsenartige Teil der Fig. 27.

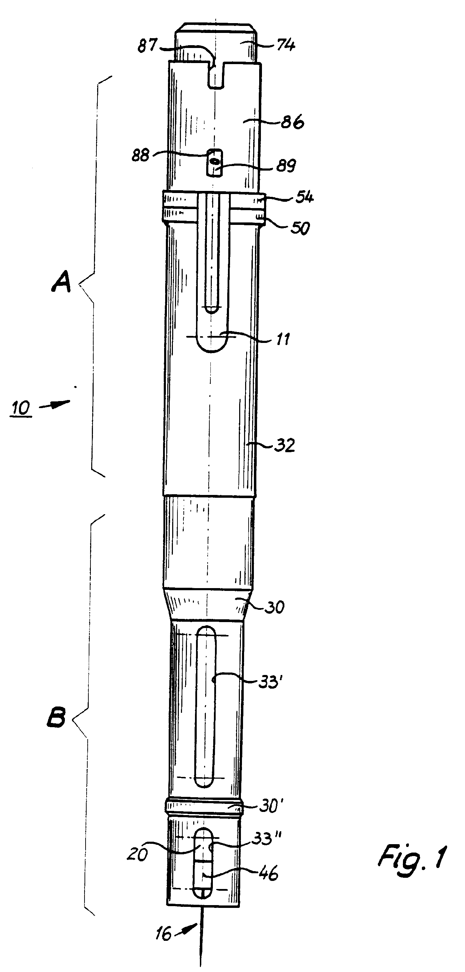

Fig. 1 zeigt ein erfindungsgemäßes Injektionsgerät 10 in der Lage nach einer Injektion. Dieses Gerät hat etwa die Form eines überdimensionierten Füllfederhalters. Seitlich hat es einen Befestigungsclip 11 der von Füllfederhaltern bekannten Form. Dieser Clip 11 dient auch als Auslöseglied für die Injektion.

4

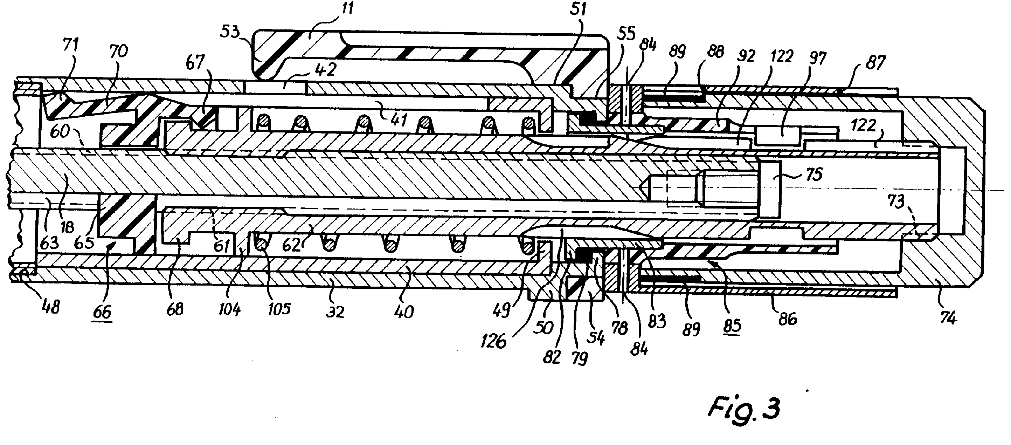

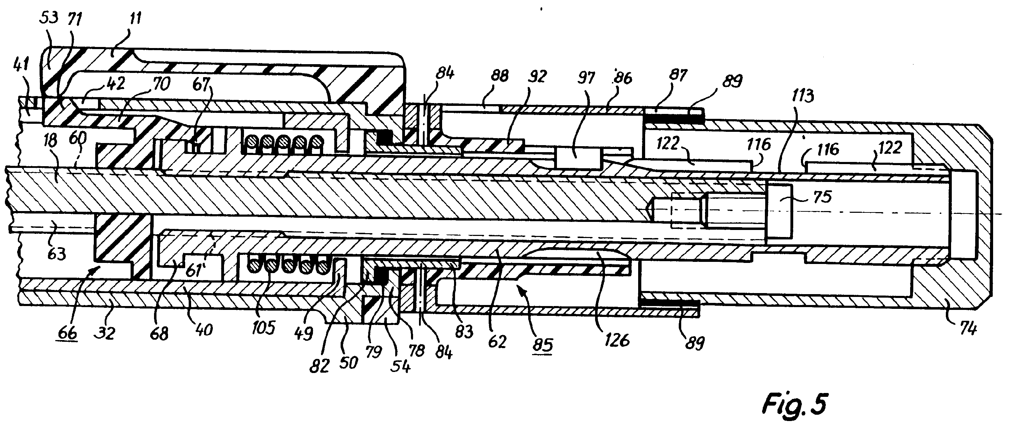

Das Injektionsgerät 10 hat ein Teil A, das zur Einstellung der zu injizierenden Flüssigkeitsmenge sowie zur Durchführung der Injektion dient und das in den Fig. 3 und 5 in stark vergrößerter Darstellung gezeigt ist, um das Verständnis zu erleichtern.

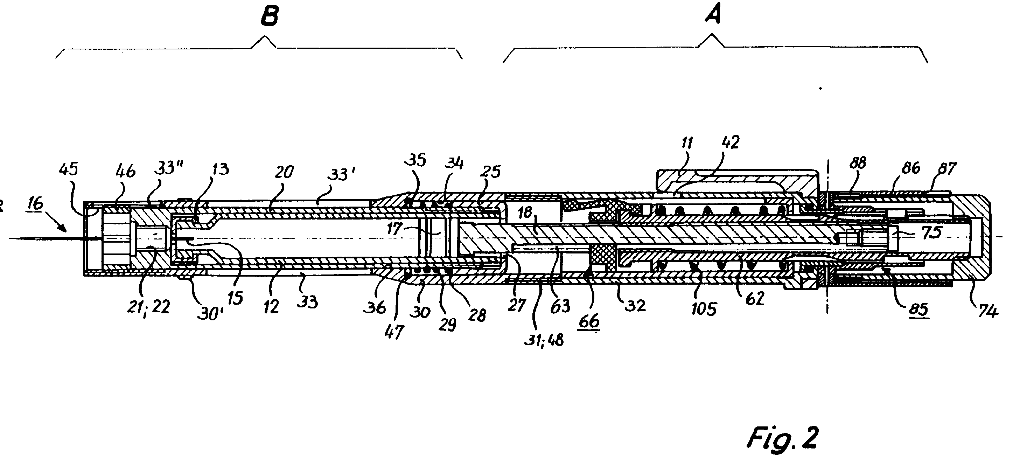

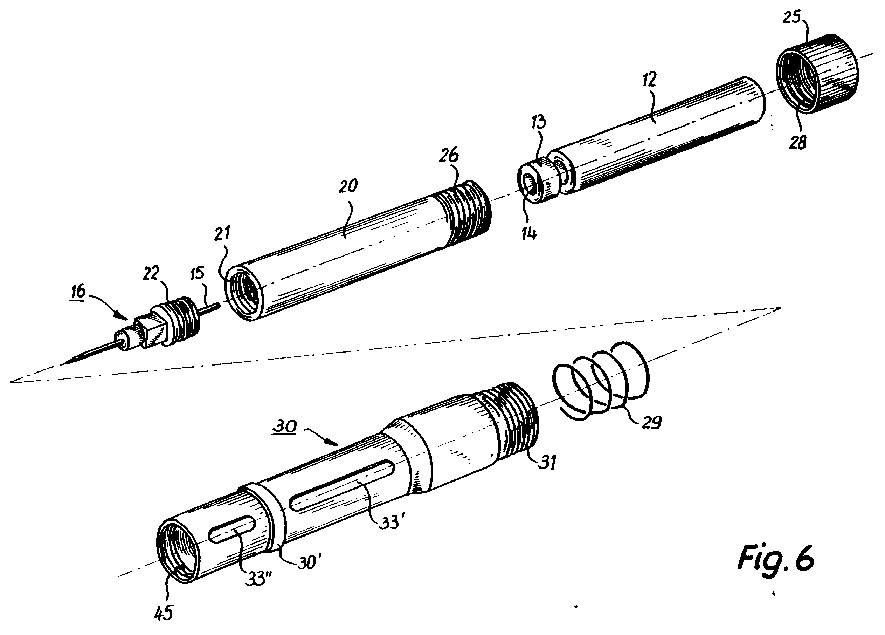

Ferner hat das Injektionsgerät 10 ein Teil B, das zur Aufnahme eines Behälters 12 (Fig. 2, 4, 6), auch Kartusche genannt, mit der zu injizierenden Flüssigkeit dient. Dieses Teil B ist in Fig. 6 in auseinandergezogener, raumbildlicher Darstellung gezeigt.

Wie man den Fig. 2,4 und 6 entnimmt, hat der Behälter 12 die Form eines länglichen, zylindrischen Glasröhrchens, das sich am proximalen, also dem Patienten zugewandten Ende verjüngt und dort mit einer aufgebördelten Aluminiumkappe 13 versehen ist, in deren Mitte sich eine dünne Gummimembran 14 (Fig. 6) befindet, die vom distalen Ende 15 einer mit 16 bezeichneten Injektionsnadel, auch Kanüle genannt, durchstoßen werden kann. Derartige Behälter mit Insulin werden z.B. von der Fa. Novo Industri AB, Dänemark, unter der Marke "Penfill" vertrieben. In dem Glasröhrchen 12 befindet sich ein verschiebbarer Kolben 17, der z. B. aus einem geeigneten Gummi hergestellt sein kann und der unter der Wirkung eines Stößels 18 (Fig. 2 - 5) in proximaler Richtung verschoben werden kann, um eine Injektion zu bewirken.

Zur Aufnahme des Behälters 12 dient eine Aufnahmehülse 20, die an ihrem proximalen Ende mit einem Innengewinde 21 versehen ist, das zur Aufnahme eines entsprechenden Außengewindes 22 der Injektionsnadel 16 dient. Da nämlich der Behälter 12 Flüssigkeit für mehrere Injektionen enthält, z.B. 100 Einheiten Insulin, sollte die Nadel 16 nach jeder Injektion gegen eine neue, sterile Nadel ausgetauscht werden, und dies geschieht durch Einschrauben einer neuen Nadel 16 in das Gewinde 21, wobei die Gummimembran 14 (Fig. 6) jeweils nach dem herausziehen des distalen Nadelendes 15 wieder einen dichten Verschluß bildet.

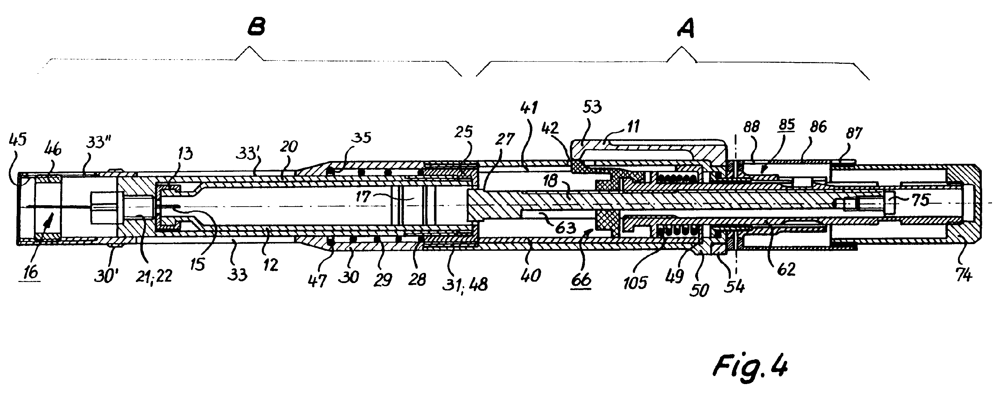

Zum Verschließen des distalen Endes der Außenhülse 20 dient eine außen gerändelte Überwurfkappe 25, die auf ein Außengewinde 26 am distalen Ende der Aufnahmehülse 20 aufgeschraubt werden kann. Diese Überwurfkappe 25 ist mit einer zentralen Ausnehmung 27 (Fig. 2 und 4) versehen, durch welche der Stößel 18 unbehindert durchdringen kann. Die Überwurfkappe 25 bildet an ihrem proximalen Ende eine Schulter 28, welche als Widerlager für eine Rückstellfeder 29 dient : Die Aufnahmehülse 20 ist aus einem durchsichtigen Kunststoff hergestellt, um eine visuelle Beobachtung des Behälters 12 zu ermöglichen, und um erkennen zu können, wie groß der Flüssigkeitsvorrat in diesem noch ist.

Ein wichtiges Sicherheitsmerkmal der vorliegenden Erfindung ist, daß die Aufnahmehülse 20 sehr genau an die Behälter (Kartuschen) 12 der einzelnen Hersteller angepaßt werden kann. Dadurch wird verhütet, daß irrtümlich ein falscher Behälter mit einer höheren Dosierung verwendet wird, was beim Patienten z.B. zu einer Überdosierung mit Insulin mit den bekannten schädlichen Folgen (Koma) führen könnte.

Zur verschiebbaren Aufnahme der Aufnahmehülse 20 dient das vordere, proximale Gehäuseteil 30, das an seinem distalen Ende mit einem Außengewinde 31 zur Verbindung mit dem hinteren, distalen Gehäuseteil 32 versehen ist. Es hat Fenster 33, 33', 33", durch welche der Inhalt des Behälters 12 beobachtet werden kann. Das vordere Gehäuseteil 30 kann aus Metall, z.B. Aluminium, oder aus einem geeigneten Kunststoff, z.B. Polypropylen, hergestellt werden. Im Bereich seines proximalen Endes ist es mit einem Tastring 30' versehen, z.B. für blinde Diabetiker. Wie die Fig. 2 und 4 zeigen, hat das vordere Gehäuseteil 30 in seinem distalen Bereich einen zylindrischen Hohlraum 34 größeren Durchmessers, der über eine Ringschulter 35 in einen zylindrischen Hohlraum 36 kleineren Durchmessers übergeht. Der Durchmesser des Hohlraums 36 ist dabei nur geringfügig größer als der Außendurchmesser der zylindrischen Aufnahmehülse 20, so daß letztere im Hohlraum 36 axial verschoben werden kann. Die Feder 29 liegt im montierten Zustand zwischen der Ringschulter 28 an der Überwurfkappe 25 und der Ringschulter 35 und beaufschlagt folglich die Aufnahmehülse 20 in distaler Richtung. In der Lage des Geräts nach einer Injektion ist die Feder 29 gespannt, wie das Fig. 2 zeigt, und der Stößel 18 liegt mit Vorspannung gegen den Kolben 17 an und verhindert, daß sich die Feder 29 entspannt. Wird der Stößel 18 zurückgezogen , wie das Fig. 4 zeigt, so entspannt sich die Feder 29 und drückt die Aufnahmehülse 20 in distaler Richtung bis zum Anschlag gegen eine im hinteren Gehäuseteil 32 befestigte zylindrische Hülse 40. Die Hülse 40 hat auf dem größten Teil ihrer Längserstreckung einen Längsschlitz 41, der zur Längsführung des Stößels 18 dient und der mit einer fensterartigen Öffnung 42 im hinteren, distalen Gehäuseteil 32 fluchtet. - In einem Innengewinde 45 am proximalen Ende des vorderen Gehäuseteils 30 ist eine Einstellmutter 46 eingeschraubt, welche zur Einstellung der Einstichtiefe der Nadel 16 dient.

Bei der Montage des B-Teils wird zunächst ein neuer Behälter 12 in der in Fig. 6 dargestellten Lage in die Aufnahmehülse 20 eingeschoben, und die Überwurfkappe 25 wird dann auf das Außengewinde 26 aufgeschraubt. Anschließend wird mittels einer (nicht dargestellten) Hilfsvorrichtung, welche die Injektionsnadel 16 steril umgibt, diese Nadel mit ihrem Außengewinde 22 in das Innengewinde 21 der Aufnahmehülse 20 eingeschraubt, wobei das distale Nadelende 15 die Membran 14 durchstößt und in die Flüssigkeit im Behälter 12 eindringt.

Anschließend wird die Aufnahmehülse 20 durch das distale Ende in das vordere Gehäuseteil 30 eingeführt, in dem sich bereits die Feder 29 befindet. Zum Schutz der Injektionsnadel 16 kann dieses Gehäuseteil 30 an seinem proximalen Ende mit einer Schutzhülse oder mit einer Schutzkappe versehen sein.

Nachdem das vordere Gehäuseteil 30 so geladen worden ist, kann es mit seinem Gewinde 31 in ein entsprechendes Gewinde 48 des hinteren Gehäuseteils 32 eingeschraubt werden und ist dann z.B. für mehrere Injektionen nach der sogenannten intensivierten Insulintherapie bereit, d.h. aus dem Behälter 12 können - je nach dem Insulinbedarf des Patienten - mehrere Injektionen mit vorgewählter Dosierung erfolgen. Wie dies geschieht, ergibt sich aus der nachfolgenden Beschreibung des A-Teils.

Hierbei soll auch noch erwähnt werde, daß die Feder 29 mit ihrem distalen Ende in einen Einstich 47 des Gehäuseteils 30 eingehängt ist, um zu verh indern, daß sie im Gebrauch verlorengeht. Diese Feder 29 ist mit großem Vorteil eine Feder mit einer sehr niedrigen Federkonstanten, die also sehr weich ist. Im gespannten Zustand, in dem die Aufnahmehülse 20 mit ihrem proximalen Ende gegen die Einstellmutter 46 anliegt, ist die Feder 29 - wie dargestellt - nicht voll zusammengepreßt, und ihre Federkraft in diesem Zustand liegt bevorzugt um etwa 20 g, maximal bei 30 g. Dies erscheint wichtig für eine einwandfreie Funktion des beschriebenen Injektionsgeräts 10.

Im hinteren Gehäuseteil 32 ist, wie bereits beschrieben, die Hülse 40 befestigt, deren in axialer Richtung verlaufender Längsschlitz 41 mit dem Fenster 42 des Gehäuseteils 32 fluchtet, d.h. durch dieses Fenster 42 kann man in das Innere der Hülse 40 schauen. Die Hülse 40 ist an ihrem distalen Ende mit einem nach innen ragenden Bund 49 versehen.

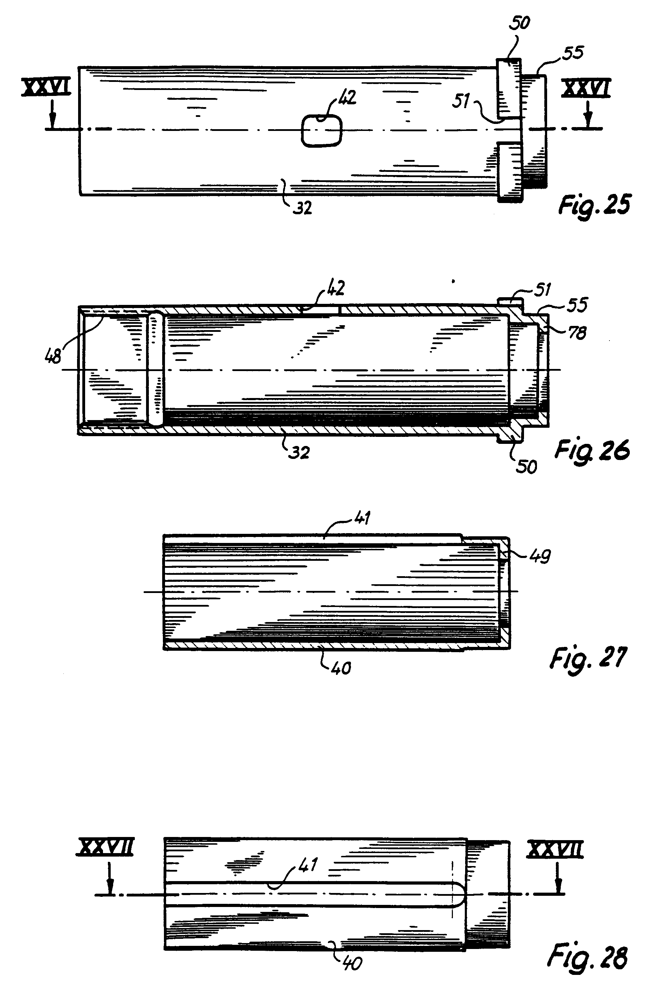

Auf der außenseite des Gehäuseteils 32 und nahe bei dessen distalem Ende ist ein Ring 50 ausgebildet, vgl. die Fig. 25 und 26. Er hat eine Unterbrechung 51, die, wie in Fig. 25 dargestellt, auf derselben Mantellinie liegt wie das Fenster 42. Diese Unterbrechung dient zur Sicherung des Clips 11 gegen Drehung. Hierzu ragt der Clip 11 in diese Unterbrechung 51, wie das z.B. Fig. 3 zeigt. Der Clip 11 ist mit einem nach innen ragenden Vorsprung 53 versehen, welcher im montierten Zustand dem Fenster 42 gegenüberliegt, vgl. die Fig. 2 - 5. Dieser Vorsprung 53 dient, wie später beschrieben wird, zur Auslösung des Injektionsvorgangs.

Der Clip 11, der z.B. als Spritzgußteil aus Polyamid ausgebildet sein kann, hat einen Haltering 54, welcher gegen den Ring 50 des Gehäuseteils 32 anliegt und einen zylindrischen Abschnitt 55 des hinteren Gehäuseteils 32 umschließt.

Im Inneren des hinteren Gehäuseteils 32 sind Bauteile befestigt, die sozusagen das Gehirn dieses Geräts sind und deren Funktion es ist, bei diesem Gerät Fehlbedienungen auszuschließen. Solche Fehlbedienungen können, wie bereits erläutert, bei der Insulininjektion zu gesundheitlichen Schäden oder auch zum Koma führen.

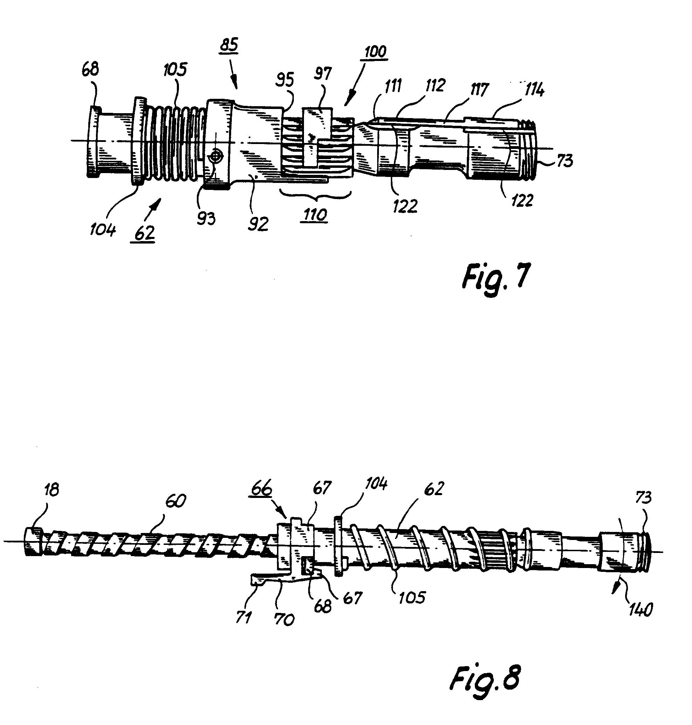

Der Stößel 18 hat auf seiner Außenseite ein Verstellgewinde 60, dessen Form (Steilgewinde) in Fig. 8 raumbildlich dargestellt ist. Dieses Gewinde 60 ist in einem entsprechenden Innengewinde 61 einer Verstellhülse 62 geführt. Dieses Innengewinde 61 ist in den Fig. 2 - 5, 9, 11 und 14A dargestellt.

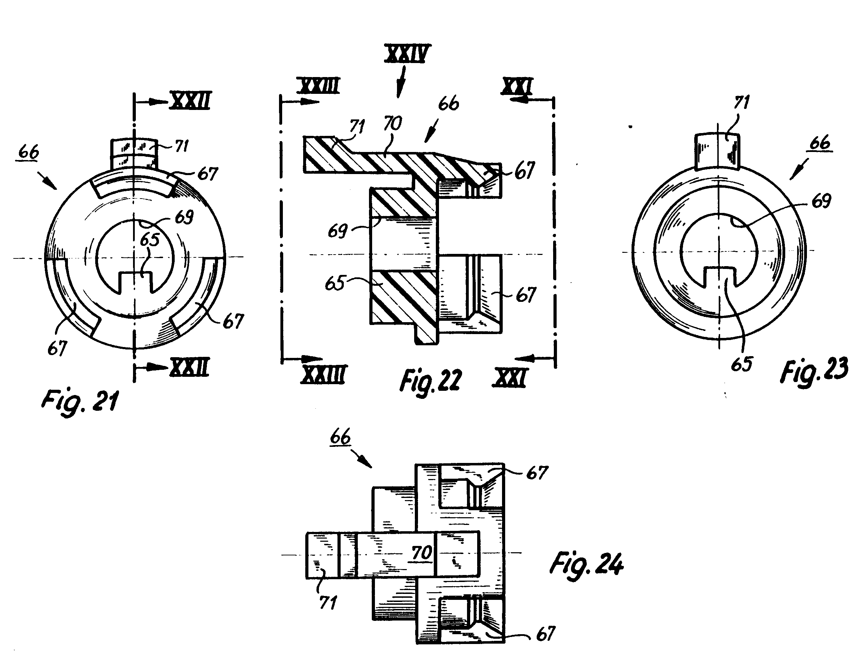

Ferner hat der Stößel 18 auf seiner Außenseite eine sich in axialer Richtung erstreckende Längsnut 63, die in den Fig. 2 bis 5 dargestellt ist und sich, ebenso wie das Außengewinde 60, vom distalen Ende des Stößels 18 bis fast zu seinem proximalen Ende erstreckt. In diese Längsnut 63 ragt ein radial nach innen ragender Vorsprung 65 eines Führungsglieds 66, das in den Fig. 21 - 24 in stark vergrößertem Maßstab dargestellt ist.

Das Führungsglied 66 hat mehrere Funktionen. Auf seiner distalen Seite ist es mit drei krallenartigen Vorsprüngen 67 versehen, die über einen Ringbund 68 am proximalen Ende der Verstellhülse 62 hinübergreifen und hinter diesem Ringbund 68 eingerastet sind. Hierdurch ist das Fährungsglied 66 mit der Verstellhülse 62 frei drehbar verbunden, kann aber axiale Kräfte von ihr bzw. auf sie übertragen. Zweckmäßig ist das Führungsglied 66 aus einem elastischen Kunststoff, z.B. einem Polyamid, ausgebildet, und deshalb können bei der Montage die krallenartigen Vorsprünge 67 auf den Ringbund 68 aufgeclipst werden. Die Innenbohrung 69 des Führungsglieds 66 ist glatt, so daß das Führungsglied auf dem Stößel 18 gleiten kann.

Ferner hat das Fährungsglied 66 auf seiner von den Vorsprüngen 67 abgewandten Seite ein federndes Rastglied 70, das an seinem freien Ende mit einer Rastnase bzw. einem Rastvorsprung 71 versehen ist. Die Rastnase 71 ist in der Längsnut 41 der Hülse 40 axial geführt und verhindert dadurch eine Drehung des Führungsgliedes 66 und des in ihm axial geführten Stößels 18, so daß sich letzterer relativ zum hinteren Gehäuseteil 32 nicht verdrehen kann. Wird die Verstellhülse 62 verdreht, so bewirkt dies, daß der Stößel 18 entweder aus der Verstellhülse 62 herausgedreht wird (dann wird die in Fig. 8 dargestellte Anordnung insgesamt länger), oder daß er in die Verstellhülse 62 hineingedreht wird (dann wird die in Fig. 8 dargestellte Anordnung kürzer). Da die Länge der in Fig. 8 dargestellten Anordnung aus Stößel 18 und Verstellhülse 62 die Injektionsmenge bestimmt, stellt dies also das Prinzip der Einstellung der Injektionsdosis dar, wobei hierzu auf dem Außengewinde 73 am distalen Ende der Verstellhülse 62 ein Betätigungsknopf 74 befestigt ist. Dieser Betätigungsknopf 74 dient einmal zum Spannen des Injektionsgeräts 10 vor der Injektion, und zum anderen dazu, im gespannten Zustand die zu injizierende Flüssigkeitsmenge einzustellen, wie das nachfolgend ausführlich beschrieben wird.

Um zu verhindern, daß der Stößel 18 ganz aus der Verstellhülse 62 herausgedreht werden kann, ist in das distale Ende des Stößels 18 - nach dessen Montage in der Verstellhülse 62 - eine Sicherungsschraube 75 eingeschraubt, die wie dargestellt als axialer Anschlag dient. Auch am proximalen Ende des Stößels 18 ist, wie dargestellt, ein solcher axialer Anschlag vorgesehen.

Die Rastnase 71 gleitet, wie in den Fig. 2 - 5 dargestellt, in der Längsnut 41 der Hülse 40. Gelangt sie beim Spannen des Injektionsgeräts 10 (mittels des Betätigungsknopfes 74) zum Fenster 42, so rastet sie in dieses ein, wie das in den Fig. 5 und 4 dargestellt ist. Durch Drücken auf den Clip 11 kann sie wieder aus dem Fenster 42 ausgerastet werden, wodurch der Injektionsvorgang ausgelöst wird.

Wie in Fig. 26 dargestellt, hat das hintere Gehäuseteil 32 an seinem distalen Ende einen nach innen ragenden Bund 78, und an diesem ist - über einen zwischengeschalteten O-Ring 79 - der Bund 82 einer Befestigungshülse 83 abgestützt, welch letztere in distaler Richtung durch die vom Bund 78 freigelassene Öffnung ragt. Auf dieser Hülse 83 sind mittels zweier Schrauben 84 eine Sperrklinke 85 und ein Nullstellglied 86 befestigt. Wird das Nullstellglied 86 verdreht, so wird mit ihm die Sperrklinke 85 und die Buchse 83 verdreht, wobei der O-Ring 79 als Rutschkupplung zwischen der Buchse 83 und dem hinteren Gehäuseteil 32 wirkt und normalerweise diese Teile relativ zum hinteren Gehäuseteil 32 festhält, also an einer Drehung hindert.

Wie aus den Fig. 1 - 5 hervorgeht, ist das Nullstellglied 86 ein dünnwandiges, zylindrisches Teil, das bei nicht gespanntem Injektionsgerät 10 (Fig. 3) den Betätigungsknopf 74 mit geringem Abstand umgibt, dabei aber eine Grifffläche an diesem freiläßt. Es hat zwei Fesnter, nämlich ein Fenster 87 an seinem distalen Ende und ein Fenster 88 im Bereich seines proximalen Endes. Der Betätigungsknopf 74 trägt eine diesen Fenstern zugeordnete Einstellskala 89, welche mit Indicia für die eingestellte Injektionsmenge versehen ist. Z.B. ist in Fig. 1 im Fenster 88 das Indicium "Null" der Skala 89 sichtbar. Dabei ist, vgl. Fig. 3, die Skala 89 im nicht gespannten Zustand durch das Fenster 88 ablesbar, im gespannten Zustand dagegen (vgl. Fig. 5) durch das Fenster 87. Da die Einstellung der Injektionsmenge nur im gespannten Zustand (Fig. 4 und 5) erfolgen kann, wobei die Ablesung durch das Fenster 87 erfolgt, ist es wichtig, daß der Patient auch nach der Injektion noch ablesen kann, welche Menge er gerade injiziert hat. Dies wird durch die beiden Fenster 87, 88 in Verbindung mit der Skala 89 in sehr eleganter und einfacher Weise erreicht und stellt in der Praxis ein wesentliches Sicherheitsmerkmal dar, das bisher bei Geräten dieser Gattung fehlte. Anders gesagt, konnte bei bekannten Geräten zu diesem Zweck nach dem Einspritzvorgang die Injektionsdosis nicht mehr abgelesen werden.

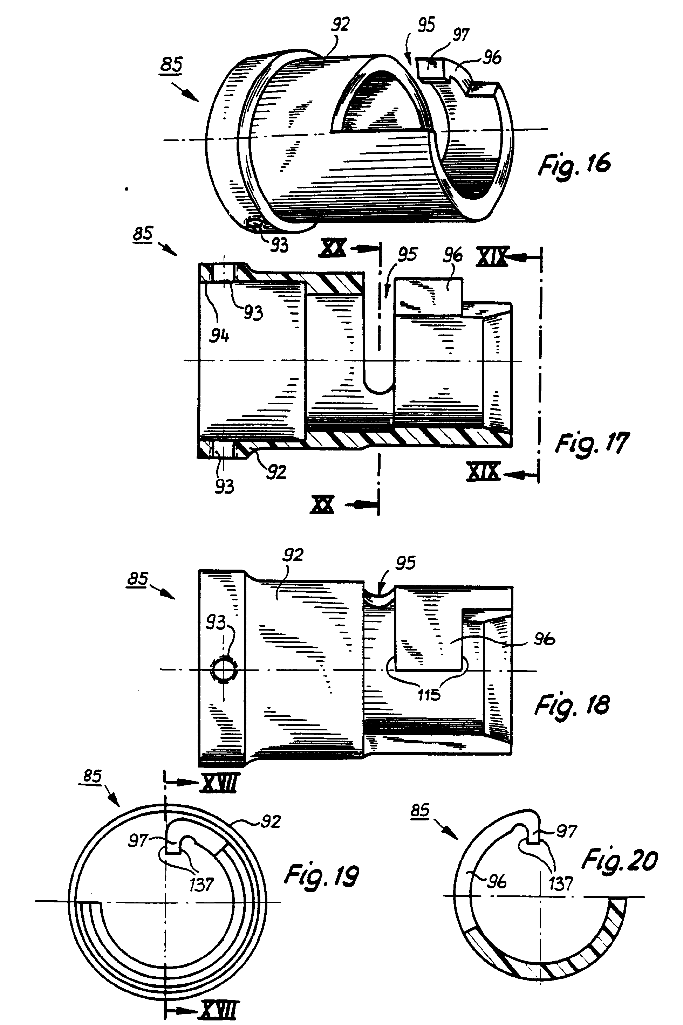

Die Sperrklinke 85 befindet sich im ungespannten Zustand im Inneren des Betätigungsknopfes 74 und liegt im wesentlichen konzentrisch zu diesem und dem Nullstellungsglied 86. Sie ist in den Fig. 16 - 20 in ihren Einzelheiten dargestellt, wobei die raumbildliche Darstellung nach Fig. 16 die Erfassung aller wesentlichen Einzelheiten ermöglicht.

Das Sperrklinkenteil 85, welches aus einem elastischen Kunststoff, z.B. einem Polyamid, besteht, hat in seinem proximalen Bereich einen zylindrischen Grundkörper 92, der mit zwei diametral gegenüberliegenden Gewindebohrungen 93 für die Befestigung der Schrauben 84 versehen ist und eine Ausdrehung 94 zur passenden Aufnahme der Befestigungshülse 83 hat, vgl. z.B. Fig. 5. Axial etwa in ihrer Mitte hat die Sperrklinkenanordnung 85 eine senkrecht zur Achsrichtung verlaufende Nut 95, die ein federnd auslenkbares Rastglied 96 abtrennt, das, wie dargestellt, etwa 120° lang sein kann und am freien Ende mit einem nach innen ragenden Rastvorsprung 97 versehen ist. Wie aus Fig. 20 besonders gut hervorgeht, ist das Rastglied 96 mit seinem Rastvorsprung 97 elastisch und radial nach außen auslenkbar. Die axiale Erstreckung des Rastglieds 96 und des Rastvorsprungs 97 ist, wie in den Fig. 16 - 18 dargestellt, relativ kurz und beträgt etwa 30 % der Gesamtlänge der Sperrklinkenanordnung 85.

Der Rastvorsprung 97 wirkt, wie das Fig. 7 zeigt, mit entsprechenden Steuerausnehmungen 100 auf der äußeren Oberfläche der Verstellhülse 62 zusammen. Hierbei ist darauf hinzuweisen, daß selbstverständlich auch eine kinematische Umkehrung möglich ist in der weise, daß die Steuerausnehmungen auf der Innenseite der Verstellhülse 62 vorgesehen werden, wobei dann die Sperrklinkenanordnung 85 auf der Innenseite der Verstellhülse 62 angeordnet werden müßte. Aus Gründen einer kompakten Bauform wird aber die dargestellte Ausführungsform bevorzugt. Fig. 7 zeigt in raumbildlicher Darstellung, wie das Sperrklinkenteil 85 die Verstellhülse 62 umgibt. Im Betrieb führen das Sperrklinkenteil 85 und die Verstellhülse 62 relativ zueinander sowohl axiale wie Rotationsbewegungen aus.

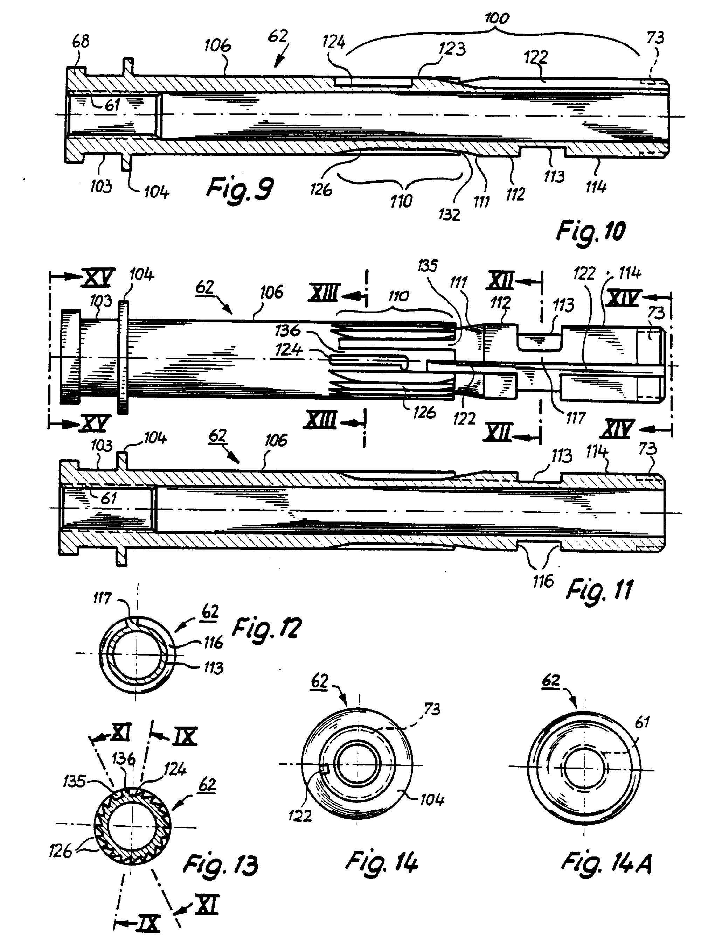

Die Fig. 9 - 15 zeigen die genaue Ausbildung eines bevorzugten Ausführungsbeispiels der Verstellhülse 62. Am proximalen Ende befindet sich der Ringbund 68 und das Innengewinde 61. Am distalen Ende befindet sich das Außengewinde 73. Auf den Ringbund 68 folgt ein zylindrischer Abschnitt 103 kleineren Durchmessers und auf ihn ein Ringbund 104, der als Widerlager für eine Spannfeder 105 dient. Die Spannfeder 105 ist in Fig. 8 im entspannten Zustand und in den Fig. 4, 5 und 7 in gespanntem Zustand dargestellt. Sie dient als Energiespeicher für den Injektionsvorgang. Ihr anderes Ende liegt gegen den Bund 49 der Hülse 40 an, wie das die Fig. 2 - 5 zeigen.

Auf den Ringbund 104 folgt in distaler Richtung ein zylindrischer Abschnitt 106, der denselben Durchmesser hat wie der zylindrische Abschnitt 103. An ihn schließt sich der Abschnitt mit den Steuerausnehmungen 100 an.

Die Steuerausnehmungen 100 haben folgende wichtige Funktionen:

a) Sie lassen ein Spannen des Injektionsgeräts 10 nur zu, wenn ein bestimmter Wert der Skala 89 im Fesnter 88 angezeigt wird. Beim Ausführungsbeispiel ist dies der in Fig. 1 angezeigte Wert "Null". Nur in dieser relativen Drehstellung zwischen Nullstellungsglied 86 und Betätigungsknopf 74 ist ein Spannen des Injektionsgerät überhaupt möglich, in allen anderen Drehstellungen dagegen gesperrt.

b) Die Steuerausnehmungen 100 lassen ferner ein Auslösen des Geräts in eben derselben Nullstellung nicht zu. Dies ist wichtig, da in der Nullstellung, und nur in der Nullstellung, das Injizieren einer kleinen Flüssigkeitsmenge nicht verhindert werden könnte, d.h. hier würde die Skalenanzeige nicht mit den tatsächlichen Verhältnissen übereinstimmen.

c) Die Steuerausnehmungen 100 lassen ferner in der gespannten Stellung ein Einstellen der gewünschten Injektionsmenge zu, aber nur bis zu einem vorgegebenen Höchstwert, z.B. nur bis 17 Einheiten.

d) Die Steuerausnehmungen 100 lassen ferner ein Auslösen einer Injektion aus allen Einstellungen zu, bei denen die Injektionsmenge einen bestimmten Mindestwert überschreitet.

e) Die Steuerausnehmungen 100 begrenzen die einstellbare Injektionsmenge nach oben.

All dies wird erreicht durch Zusammenwirken der Steuerausnehmungen 100 mit dem Rastvorsprung 97 (Fig. 16 - 20).

Die Steuerausnehmungen 100 haben einen Abschnitt 110, der hauptsächlich der Einstellung der Einspritzmenge dient und der im Querschnitt in Fig. 13 dargestellt ist; daran schließt an ein erster Sperrabschnitt 111, dann ein zylindrischer Abschnitt 112, ein Abschnitt 113 kleineren Durchmessers für die Nullstellung im entspannten Zustand, und schließlich wieder ein zylindrischer Abschnitt 114, der an seinem distalen Ende mit dem Außengewinde 73 versehen ist.

Im entspannten Zustand, wie er in den Fig. 2 und 3 dargestellt ist, befindet sich der Rastvorsprung 97 im Abschnitt 113 und füllt diesen im wesentlichen aus, wobei seinen seitlichen Flanken 115 (Fig. 18) die seitlichen Flanken 116 (Fig. 11) des Abschnitts 113 mit geringen Abständen gegenüberliegen. In dieser Stellung kann die Verstellhülse 62 relativ zum Sperrklinkenglied 85, das starr mit dem Nullstellglied 86 verbunden ist, um fast 360° verdreht werden.

Eine axial verlaufende Trennwand 117 (Fig. 10 und 12) im Abschnitt 113 wirkt als Anschlag und Begrenzung für eine solche relative Drehung und begrenzt sie auf weniger als 360°. Eine solche relative Drehung in diesem beschnitt 113 ist, wie gesagt, nur im entspannten Zustand möglich, wie ihn die Fig. 2 und 3 zeigen.

Um das Sperrklinkenglied 85 überhaupt auf diese Verstellhülse 62 aufschieben zu können, ist am distalen Abschnitt der letzteren eine axiale Längsnut 122 vorgesehen, die sich vom distalen Ende der Verstellhülse 62 bis zum distalen Ende des Abschnitts 110 erstreckt und deren Querschnittsform im oberen Teil von Fig. 9 dargestellt ist. Ihre Tiefe ist bis zum beschnitt 111 konstant, not etwa ab dem Abschnitt 111 in proximaler Richtung ab, und wird im distalen Bereich des Abschnitts 110 zu Null, vgl. Fig. 9. Auf das proximale Ende dieser Nut 122 folgt ein kurzer zylindrischer Abschnitt 123, und dann eine Ausnehmung 124, die - wie in Fig. 9 dargestellt - im Längsschnitt einen rechteckförmigen Querschnitt mit senkrechten Flanken hat.

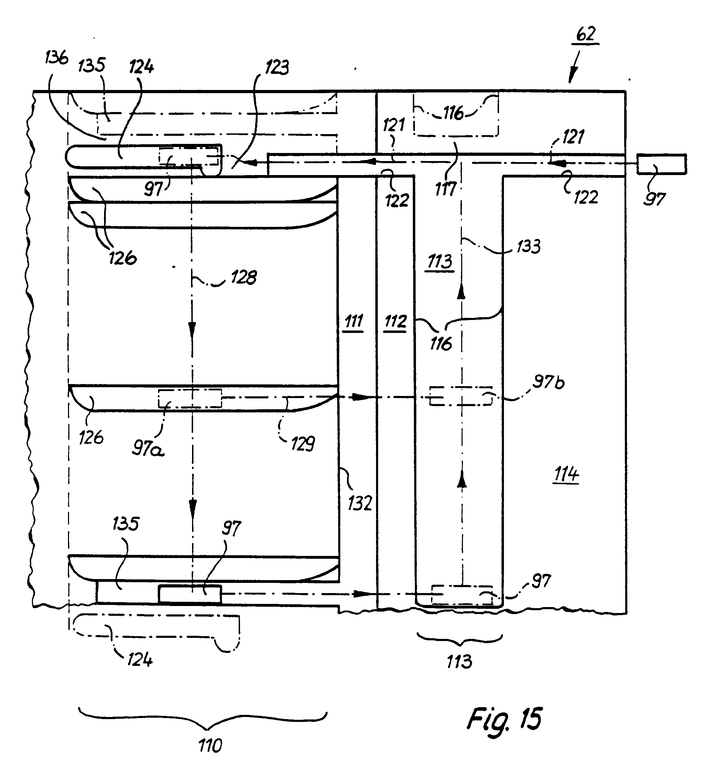

Fig. 15 zeigt einen Abschnitt der Verstellhülse 62 in Abwicklung (in developed form). Man erkennt, daß der Rastvorsprung 97 sich längs der strichpunktierten Linie 121 durch die Nut 122 nach links, also in proximaler Richtung bewegen kann. (In Wirklichkeit bewegt sich die Verstellhülse 62 in axialer Richtung relativ zum Rastvorsprung 97). Gelangt der Rastvorsprung 97 zum zylindrischen Abschnitt 123, so wird er radial nach außen ausgelenkt und gleitet dann in die Ausnehmung 124 und rastet dort ein, kann sich also dann nicht mehr in distaler Richtung bewegen. Bei diesem Vorgang wird die Feder 105 gespannt, und das Rastglied 21 rastet, wie in Fig. 5 dargestellt, in das Fenster 42 ein.

Die Stellung, in der der Rastvorsprung 97 mit der Längsnut 122 fluchtet, entspricht der Injektionsmenge Null, wie sie in Fig. 1 dargestellt ist (Anzeige "Null" im Fenster 88).

Nur in dieser Stellung ist also ein Spannen möglich, und wenn nach dem Spannen der Rastvorsprung 97 in die Ausnehmung 124 eingerastet ist, ist eine Auslösung durch Betätigung des Clips 11 zwar möglich, hat aber keine Wirkung, da dann eine axiale Bewegung des Rastvorsprungs 97 längs der strichpunktierten Linie 121 durch die Einrastung des Rastvorsprungs 97 in der Ausnehmung 124 verhindert wird. Dies verhindert ein Injektion bei der Einstellung für die Injektionsmenge Null, aus den vorstehend unter a) erläuterten Gründen.

Bezogen auf Fig. 13 folgen auf die Ausnehmung 124 in Umfangsrichtung insgesamt 18 Rastzähne 126 mit gleichen Abständen voneinander. Befindet sich der Rastvorsprung 97 in der Ausnehmung 124, so kann er nun durch Verdrehen des Einstellknopfes 74 in eine der durch die Rastzähne 126 definierten Raststellungen gebracht werden, was einer bestimmten Verdrehung der Verstellhülse 62 relativ zum Gehäuseteil 32 und damit einem Herausdrehen des Stößels 18 aus der Verstellhülse 62 entspricht, oder anders gesagt, die in Fig. 8 dargestellte Anordnung wird proportional zu dieser Verdrehung länger. Dies ist in Fig. 15 durch die strichpunktierte Linie 128 angedeutet. Kommt hierbei der Rastvorsprung z.B. in die Stellung 97a, so entspricht dies einer bestimmten Injektionsmenge, z.B. 10 IE Insulin. In dieser Stellung kann nun durch Druck auf den Clip 11 das Injektionsgerät 10 ausgelöst werden, und der Rastvorsprung 97a führt dann eine relative Gleitbewegung längs der strichpunktierten Linie 129 aus, also aus der Rastausnehmung heraus hinein in den konischen beschnitt 111 und der den zylindrischen beschnitt 112 zurück in den Abschnitt 113, wo dann dieser Rastvorsprung die Stellung 97b einnimmt. In dieser Stellung wird ein erneutes Spannen dadurch verhindert, daß der Rastvorsprung 97b dabei gegen die proximale Flanke 116 des Abschnitts 113 stößt. Sollte es zufällig gelingen, diese proximale Flanke 116 zu überwinden, so stellt die proximale Flanke 132 des konischen Abschnitts 111 das nächste Hindernis dar, das ein Spannen bis zum Einrasten ebenfalls verhindert. Auch ein blinder Diabetiker wird hierdurch daran erinnert, daß er sein Gerät noch nicht neu eingestellt hat, und daß eine Injektion nicht möglich ist. Dabei wird durch die Flanken bzw. Schultern 116 und 132 eine doppelte Sicherheit erreicht. ( Bei den Ausführungsbeispielen der Fig. 2 bis 5 ist die Flanke 132 nicht vorgesehen.) Die Rastzähne 126 ermöglichen nur ein Verstellen in Richtung zunehmender Injektionsmengen, und ihre Tiefe nimmt, wie dargestellt, in distaler Richtung ab, damit das Teil 97 seitlich herausgleiten kann. - Soll eine neue Injektion vorgenommen werden, so muß zunächst durch Verdrehung des Nullstellungsgliedes 86 und des mit ihm starr verbundenen Sperrklinkenteils 85 der Rastvorsprung 97 längs der strichpunktierten Linie 133 bis zum Anschlag gegen die Trennwand 117 verdreht werden. Dann zeigt die Skala 89 im Fenster 88 den Wert "0" an, vgl. Fig. 1, und ein neues Spannen wird möglich. Es ist darauf hinzuweisen, daß dieses Verdrehen des Nullstellungsglieds 86 keine Veränderung der Lage des Stößels 18 bewirkt.

Die Einstellung der Einspritzdosis im gespannten Zustand ist nur soweit möglich, bis der Rastvorsprung 97 eine Lage in einer Ausnehmung 135 erreicht, die durch eine Trennwand 136 von der Ausnehmung 124 getrennt ist. Diese Trennwand 136 hat senkrechte Wände und kann deshalb vom Rastvorsprung 97 nicht überwunden werden, der, wie in Fig. 19 und 20 dargestellt, ebenfalls in Umfangsrichtung durch senkrechte Flanken 137 begrenzt ist. Die Trennwand 136 stellt also einen Anschlag dar, der die Einspritzdosis nach oben begrenzt, z.B. auf 17 IE (internationale Einheiten) Insulin. Aus dieser Einstellung kann ebenfalls ausgelöst werden.

Arbeitsweise. Das B-Teil wird zunächst mit einer neuen Kartusche 12 geladen, wie das eingangs ausführlich beschrieben wurde, und es wird eine neue Nadel 16 eingesetzt. Dann wird - im ausgelösten Zustand des Geräts 10 - dadurch, daß die Verstellhülse 62 in Richtung des Pfeiles 140 (Fig. 8) gedreht wird, also dadurch, daß von der distalen Seite her gesehen der Knopf 74 entgegen dem Uhrzeigersinn verdreht wird, der Stößel 18 so weit wie möglich in die Verstellhülse 62 eingedreht. Anschließend wird das Gehäuseteil 30 mit dem Gehäuseteil 32 zusammengeschraubt. Das proximale Ende des Stößels 18 hat nun, ohne daß das Gerät gespannt ist, einen Abstand vom Kolben 17 der Kartusche 12, und dieser Abstand muß zu Null gemacht werden. Deshalb wird nun der Betätigungsknopf 74 so lange zusammen mit dem Nullstellungsglied 86 im Uhrzeigersinn (gesehen von der distalen Seite des Geräts) verdreht, bis der Stößel 18 den Kolben 17 berührt, wie das in Fig. 2 dargestellt ist. Der Benutzer merkt dies daran, daß ein Tropfen aus der Nadel 16 austritt.

Die Skala zeigt nun einen hohen Wert an, und deshalb wird als nächstes das Nullstellungsglied 86 so lange allein im Uhrzeigersinn verdreht, bis die Skala den Wert "Null" anzeigt.

Nun kann das Injektionsgerät 10 gespannt werden. hierzu wird der Betätigungsknopf 74 in distaler Richtung so weit aus dem Nullstellungsglied 86 herausgezogen, bis der Rastvorsprung 71 in das Fenster 42 des Gehäuseteils 32 einrastet. Man erhält dann die Stellung entsprechend Fig. 4.

Als nächstes wird die Injektionsdosis eingestellt. hierzu wird der Einstellknopf 74 nun - in seiner gespannten Stellung im Uhrzeigersinn (gesehen vom distalen Ende) verdreht, bis die Skala 89 die gewünschte Injektionsdosis anzeigt. Ein blinder Diabetiker kann die Injektionsdosis auch dadurch einstellen, daß er die beim Einstellen hörbaren Klickgeräusche zählt. Diese können z.B. jeweils einer IE entsprechen.

Nach dem Einstellen wird das Gerät 10 mit seinem proximalen Ende auf das gewünschte Körperteil aufgesetzt, in das injiziert werden soll, z.B. auf eine Hüfte. Dann wird der Clip 11 gedrückt, wodurch der Rastvorsprung 71 aus dem Fenster 42 herausgedrückt wird. Die gespannte Feder 105 entspannt sich nun und beschleunigt den Stößel 18 in proximaler Richtung. Dabei trifft dieser auf den Kolben 17. Da der Kolben 17 einer Bewegung in der Kartusche 12 einen Widerstand entgegensetzt, wird als erstes die Kartusche 12 mit ihrem Führungsrohr 20 entgegen der - schwachen - Gegenkraft der Rückstellfeder 29 in proximaler Richtung bewegt, wobei die Nadel 16 in den Patienten einsticht. Hierbei kommt das Führungsrohr 20 zur Anlage gegen den (verstellbaren) Anschlag 46 (dessen Lage im Fenster 33″ sichtbar ist). Nun wirkt die kraft der Feder 105 über den Stößel 18 nur noch auf den Kolben 17 und verschiebt diesen in der Kartusche in proximaler Richtung, so daß eine dieser Verschiebung - die zuvor vom Benutzer eingestellt wurde - entsprechende Flüssigkeitsmenge injiziert wird.

Nach der Injektion wird die Nadel 16 aus dem Körper des Patienten herausgezogen und gegen eine neue ausgewechselt und mit einer sterilen kappe geschützt. Anschließend wird durch Verdrehen des Nullstellungsgliedes 86 im Uhrzeigersinn die Skala wieder auf Null gebracht. Vor dem nächsten Spannen wird zuerst wieder ein Tropfen aus der Nadel 16 gepreßt, um die richtige Lage des Stößels 18 zu überprüfen.

Das erfindungsgemäße Gerät ermöglicht eine Injektion auch an Körperstellen, z.B. dem Gesäß, die bei der Selbstinjektion für normale Spritzen nicht erreichbar wären. Es ermöglicht es ferner, aus einer einzigen Kartusche eine Reihe von willkürlich dosierbaren Injektionen zu geben. Da das erfindungsgemäße Gerät recht klein ist, kann es der Patient überallhin mit sich nehmen und deshalb die intensivierte Insulintherapie z.B. auch auf Reisen fortsetzen. Durch die spezielle Gestaltung der Steuerausnehmungen 100 in Verbindung mit dem Rastvorsprung 97 wird eine praktisch narrensichere Bedienung ermöglicht. Dabei ist natürlich zu berücksichtigen, daß der Patient stets in einer Klinik im Gebrauch solcher Injektionsgeräte sorgfältig unterwiesen wird, und daß stets ein Kompromiß zwischen der Größe eines solchen Geräts und den in ihm eingebauten Funktionen getroffen werden muß.

Naturgemäß sind im Rahmen der vorliegenden Erfindung zahlreiche Abwandlungen und Modifikationen möglich.

Ein wichtiges Sicherheitsmerkmal ist auch gegeben durch die Form der Rastzähne 126 in Verbindung mit dem Sperrklinkenglied 85. In der gespannten Stellung (Fig. 4, 5) kann nämlich der Betätigungsknopf 74 befolge der Form dieser Rastzähne 126 nur in einer die Injektionsmenge vergrößerenden Richtung verstellt werden, oder anders ausgedrückt, der Weg 128 in Fig. 15 ist ebenso wie die Wege 122 und 129 eine Einbahnstrasse, die in Fig. 15 einen "Kreisverkehr" erzwingt.

Für die Sicherheit spielt auch die Rutschkupplung in Gestalt des O-Rings 79 eine wichtige Rolle, weil sie das Nullstellungsglied 86 festhält und erst bei einem relativ hohen Losbrechmoment freigibt. Dadurch wirkt das Nullstellungsglied 86 - und mit ihm das mit ihm starr verbundene Sperrklinkenglied 85 - praktisch wie ein gehäusefestes Teil, kann jedoch zur Nullstellung der Skalenanzeige nach der Injektion gut verdreht und in eine neue Nullstellung gebracht werden. Natürlich können zur Erzeugung dieses Losbrechmoments auch beliebige andere Mittel verwendet werden. Auch könnte das Nullstellungsglied zusätzlich mechanisch blockiert werden, wenn das Gerät 10 gespannt ist.

Beim vorstehend beschriebenen Ausführungsbeispiel wird die richtige Bedienung durch eine Anordnung sichergestellt, die man auch als mechanischen Computer bezeichnen könnte. Selbstverständlich könnte im Rahmen der Erfindung stattdessen auch ein Mikroprozessor verwendet werden, der dann noch weitere Eingangssignale verarbeiten könnte, z.B. den Füllstand der Kartusche 12, Vorhandensein der Nadel 16, Überprüfung der eingestellten Injektionsmenge auf Plausibilität, etc., und der nach einer Injektion eine automatische Nullstellung bewirken könnte. Ein solches Gerät wäre dann aber relativ schwer und groß , während das Gerät nach dem Ausführungsbeispiel kompakt ist und keine zusätzlichen Energiequellen wie Batterien benötigt und daher überallhin mitgenommen und auch leicht sterilisiert werden kann.

## Patentansprüche

1. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung (Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,
welch letzterer einen Anschlag (117) für den Eingriff mit dem Eingriffsglied (85) aufweist, welcher bei Eingriff des Eingriffsgliedes (85) mit dem zweiten Abschnitt (113) die relative Verdrehung zwischen dem als erstes Stellglied dienenden Spannglied (74) und dem zweiten Stellglied (86) auf einen vorgegebenen Drehwinkelbereich begrenzt.

2. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung (Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,

welch letzterer eine Steuerausnehmung (116, 122) für den Eingriff des Eingriffsglieds (85) aufweist, die bei Eingriff mit dem Eingriffsglied (85), also nach dem Injektionsvorgang, außerhalb einer vorgegebenen relativen Drehstellung zwischen Eingriffsglied (85) und Gegenglied (62) eine axiale Spannbewegung des als erstes Stellglied dienenden Spannglieds (74) sperrt.

3. Injektionsgerät nach Anspruch 2, dadurch gekennzeichnet, daß die relative Ruhestellung dem Wert Null einer zwischen Spannglied (74) und zweitem Stellglied (86) vorgesehenen Skalenanzeige (89) entspricht.

4. Injektionsgerät nach Anspruch 3, dadurch gekennzeichnet, daß der zweite Abschnitt des Gegenglieds (62) als Ringnut (113) ausgebildet ist, gegen deren einen Rand das Eingriffsglied (85), auf diese Weise eine axiale Spannbewegung des Spannglieds (74) verhindernd, anliegt, wenn das Spannglied (74) nicht gespannt ist, und daß in der vorgegebenen Winkelstellung (Fig. 9) oder im vorgegebenen Winkelstellungsbereich dieser Rand (116) eine Aussparung (122) aufweist, welche einer axialen Spannbewegung des Eingriffsglieds (85) kein Hindernis entgegensetzt.

5. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung (Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,
welcher erste Abschnitt (110) mit Ratschenzähnen (126) für den Eingriff des Eingriffsglieds (85) versehen ist, die bei Eingriff mit dem Eingriffsglied (85) eine schrittweise Verdrehung zwischen erstem und zweitem Stellglied (74 bzw. 85) und damit ein Einstellen der Injektionsdosis, insbesondere nach Gefühl oder Gehör, ermöglichen.

6. Injektionsgerät nach Anspruch 5, dadurch gekennzeichnet, daß die Form der Ratschenzähne (126) in Richtung zum zweiten Abschnitt (113) des Gegenglieds (62) so ausgebildet ist, daß diese Form bei der Injektionsbewegung des dem Bewirken eines Injektionsvorganges dienenden Organs (18, 62) ein axiales Herausgleiten des Eingriffsglieds (85) aus den Ratschenzähnen (126) ermöglicht.

7. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung (Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,
welcher erste Abschnitt (110) eine Sperrvorrichtung (124) aufweist, die bei einer vorgegebenen relativen Drehstellung zwischen Eingriffsglied (85) und Gegenglied (62) in der Spannstellung eine Injektionsbewegung des dem Bewirken eines Injektionsvorganges dienenden Organs (18, 62) durch Eingriff der Sperrvorrichtung (124) mit dem Eingriffsglied (85) blockiert.

8. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung ( Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,
welcher erste Abschnitt (110) einen Anschlag (136) für den Eingriff mit dem Eingriffsglied (85) aufweist, der durch Eingriff mit dem Eingriffsglied (85) eine Verdrehung zwischen Eingriffsglied (85) und Gegenglied (62) auf einen vorgegebenen Drehwinkelbereich beschränkt, um die einstellbare Injektionsdosis nach oben hin zu begrenzen.

9. Injektionsgerät mit einem zum Bewirken eines Injektionsvorganges dienenden, hinsichtlich seiner Länge verstellbaren Organ (18, 62), welches durch Längsverschiebung eines als erstes Stellglied dienenden Spannglieds (74) entgegen der Kraft einer Feder (105) in eine Spannstellung (Fig. 4, 5) bringbar ist, aus der es zum Bewirken eines Injektionsvorgangs freigebbar ist, um eine Wand, insbesondere einen Kolben (17), eines Behälters (12) für eine zu injizierende Flüssigkeit zu beaufschlagen,
und welches durch Verdrehung des als erstes Stellglied dienenden Spannglieds (74) zwecks Einstellung einer gewünschten Injektionsdosis hinsichtlich seiner Länge verstellbar ist,
ferner mit einem zweiten Stellglied (86) und mit einer von der Lage des ersten relativ zum zweiten Stellglied (74 bzw. 86) abhängigen Steueranordnung (62, 85), welche ein Eingriffsglied (85) aufweist, das durch die axiale Verschiebung des als erstes Stellglied dienenden Spannglieds (74) nach dem Spannen mit einem ersten Abschnitt (110) und nach dem Injektionsvorgang mit einem zweiten Abschnitt (113) eines Gegenglieds (62) selektiv in Eingriff bringbar ist,
welcher erste Abschnitt (110) mit Steuerausnehmungen (100) für den Eingriff mit dem Eingriffsglied (85) versehen ist, die bei Eingriff mit dem Eingriffsglied (85) im Zusammenwirken mit diesem eine relative Drehbewegung zwischen dem Eingriffsglied (85) und diesen Steuerausnehmungen (100) nur in einer die Injektionsdosis vergrößernden Richtung ermöglichen.

10. Injektionsgerät nach einem oder mehreren der vorhergehenden Ansprüche, dadurch gekennzeichnet, daß das zweite Stellglied (86) relativ zum als erstes Stellglied dienenden Spannglied (74) verdrehbar ist, und daß zwischen ihnerL eine SkalenarLzeige (87, 88, 89) vorgesehen ist, welcLe die relative Drehstellung zwischen ihnen anzeigt und bevor-zugt als Anzeige für die eingestellte Injektionsdosis ausgebildet ist.

11. Injektionsgerät nach einem oder mehreren der vorhergehenden Ansprüche, dadurch gekennzeichnet, daß das zweite Stellglied (86) über eine Rutschkupplung (79) mit dem Gehäuse (32) des Injektionsgeräts (10) drehbar verbunden ist.

12. Injektionsgerät nach Anspruch 11, dadurch gekennzeichnet, daß die Rutschkupplung (79) dazu ausgebildet ist, dem Stellglied (86) erst oberhalb eines auf dieses wirkenden vorgegebenen Mindest-Drehmoments eine Drehung relativ zum Gehäuse (32) zu ermöglichen.

13. Injektionsgerät nach einem oder mehreren der vorhergehenden Ansprüche, dadurch gekennzeichnet, daß das zweite Stellglied (86) das Spannglied (74) in dessen gespanntem Zustand - und bevorzugt auch in dessen ungespanntem Zustand - mindestens teilweise umgibt, um ein Einklemmen der Finger oder dergleichen des Benutzers zwischen zweitem Stellglied (86) und als erstes Stellglied dienenden Spannglied (74) zu vermeiden.

14. Injektionsgerät nach einem oder mehreren der vorhergehenden Ansprüche, dadurch gekennzeichnet, daß am Umfang des als erstes Stellglied dienenden Spannglieds (74) Skalenmarkierungen (89) vorgesehen sind, und daß das zweite Stellglied (86) eine erste Aussparung (87) aufweist, in welcher sich im gespannten Zustand des Spannglieds (74) der durch dessen Verstellung eingestellte Anzeigewert der Skalenmarkierung (89) befindet.

15. Injektionsgerät nach einem oder mehreren der vorhergehenden Ansprüche, dadurch gekennzeichnet, daß am Umfang des Spannglieds (74) Skalenmarkierungen (89) vorgesehen sind, und daß das zweite Stellglied (86) eine zweite Aussparung (88) aufweist, in welcher sich im ungespannten Zustand des Spannglieds (74) der durch dessen Verstellung eingestellte Anzeigewert der Skalenmarkierung (89) befindet.

16. Injektionsgerät nach einem oder mehreren der vorhergehenden Ansprüche, dadurch gekennzeichnet, daß das Gegenglied (62) als Bestandteil des dem Bewirken eines Injektionsvorganges dienenden Organs (18, 62) ausgebildet oder mit diesem verbunden ist.

17. Injektionsgerät nach Anspruch 16, dadurch gekennzeichnet, daß das in seiner Länge verstellbare Organ (18, 62) eine Gewindespindel (18) aufweist, und daß das Gegenglied (62) ein Innengewinde (61) für die Führung dieser Gewindespindel (18) aufweist.

18. Injektionsgerät nach Anspruch 16 oder 17, dadurch gekennzeichnet, daß das Gegenglied (62) an seinem der Gewindespindel (18) zugewandten Ende mit einem bevorzugt als Kunststoffteil ausgebildeten Führungsglied (66) versehen ist, welches drehbar an dem der Gewindespindel (18) zugewandten Ende (104) des Gegenglieds (62) befestigt ist, in eine Längsnut (63) der Gewindespindel (18) eingreift (65), und seinerseits im Gehäuse (30, 32) des Injektionsgeräts (10) in Längsrichtung geführt (41) ist, wobei das Führungsglied (66) bevorzugt auf das zugeordnete Ende (104) des Gegenglieds (62) aufgeclipst ist.

19. Injektionsgerät nach Anspruch 18, dadurch gekennzeichnet, daß das Führungsglied (66) mit einem Fortsatz (70, 71) im Gehäuse (30, 32) des Injektionsgeräts (10) in Längsrichtung geführt ist, und daß dieser Fortsatz (70, 71) als Rastglied ausgebildet ist, welches in der Spannstellung (Fig. 4, 5) in eine entsprechende Rastausnehmung (42) eingreift und dadurch das dem Bewirken eines Injektionsvorgangs dienende Organ (18, 62) in dieser Spannstellung (Fig. 4, 5) festhält.

20. Injektionsgerät nach Anspruch 19, dadurch gekennzeichnet, daß die Rastausnehmung (42) als Durchbrechung des Gehäuses (32) des Injektionsgeräts (10) ausgebildet ist, und daß ein von außen betätigbares Auslöseorgan, insbesondere ein Befestigungsclip (11), zur Freigabe des Rastglieds (70, 71), also zur Auslösung des Injecktionsvorgangs, vorgesehen ist.

21. Injektionsgerät nach einem oder mehreren der vorhergehenden Ansprüche, dadurch gekennzeichnet, daß der Behälter (12, 20) für die zu injizierende Flüssigkeit gegen die Wirkung einer Rückstellfeder (29) bis zu einem Anschlag (46) verschiebbar ist, um bei der Freigabe des zum Bewirken eines Injektionsvorganges dienenden Organs (18, 62) zumächst eine Verschiebung dieses Behälters (12, 20) bis zu diesem Anschlag (46) und dadurch eine Bewegung der Injektionskanüle (16) in Einstechrichtung und anschließend eine Beaufschlagung des Kolbens (17) in diesem Behälter (12, 20) und dadurch einen Injektionsvorgang zu bewirken.

## Claims

1. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected, and which is adjustable in length for setting a desired injection dose by rotating the cocking element (74) serving as the first setting element, further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process, which latter portion comprises a stop (117) for engagement with the engagement element (85), which upon engagement of the engagement element (85) with the second portion (113) limits the relative rotation between the cocking element (74) serving as the first setting element and the second setting element (86) to a predetermined rotational angle range.

2. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected, and which is adjustable in length for setting a desired injection dose by rotating the cocking element (74) serving as the first setting element, further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process, which latter portion comprises a control recess (116, 122) for engagement with the engagement element (85), which upon engagement with the engagement element (85), i.e. after the injection process, blocks an axial cocking motion of the cocking element (74) serving as the first setting element, outside a predetermined relative rotational position between the engagement element (85) and the counterpart element (62).

3. Injection device according to claim 2, characterised in that the relative position of repose corresponds to the zero value of a scale indicator (89) provided between the cocking element (74) and the second setting element (86).

4. Injection device according to claim 3, characterised in that the second portion of the counterpart element (62) is embodied as an annular groove (113), against one edge of which the engagement element (85) rests, in this way preventing an axial cocking motion of the cocking element (74), if the cocking element (74) is not cocked, and in that in the predetermined angular position (Fig.9) or the predetermined angular position range this edge (116) has a recess (122) which presents no hindrance to an axial cocking motion of the engagement element (85).

5. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected,
and which is adjustable in length for setting a desired injection dose by rotating the cocking element (74) serving as the first setting element,
further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process,
which first portion (110) is provided with ratchet teeth (126) for engagement with the engagement element (85), which ratchet teeth, upon engagement with the engagement element (85), enable an incremental rotation between the first and second setting element (74 and 85 respectively), and hence enable adjustment of the injection dose, in particular by feel and/or by hearing.

6. Injection device according to claim 5, characterised in that the shape of the ratchet teeth (126) in the direction toward the second portion (113) of the counterpart element (62) is embodied such that upon the injection motion of the mechanism (18, 62) used to effect the injection process, this shape enables an axial sliding of the engagement element (85) out of the ratchet teeth (126).

7. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected,
and which is adjustable in length for setting a desired injection dose by rotating the cocking element (74) serving as the first setting element,
further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process,
which first portion (110) comprises a blocking device (124) which in the cocked position, at a predetermined relative rotational position between the engagement element (85) and the counterpart element (62), blocks an injection motion of the mechanism (18, 62) used to effect an injection process by causing the blocking device (124) to engage with the engagement element (85).

8. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected,
and which is adjustable in length for setting a desired injection dose by rotating the cocking element (74) serving as the first setting element,
further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process,
which first portion (110) comprises a stop (136) for engagement with the engagement element (85) that by engagement with the engagement element (85) limits rotation between the engagement element (85) and the counterpart element (62) to a predetermined rotational angle range, in order to place an upper limit on the injection dose that can be set.

9. Injection device, having a mechanism (18, 62) used to effect an injection process, the length of which mechanism is variable and which can be moved by longitudinal displacement of a cocking element (74) serving as a first setting element counter to the force of a spring (105) into a cocked position (Figs. 4, 5) from which it can be released in order to effect an injection process, so as to act upon a wall, in particular a piston (17), of a container (12) containing fluid to be injected,

and which is adjustable in length for setting a desired injection dose by rotating the cocking element (74) serving as the first setting element,

further having a second setting element (86) and a control arrangement (62, 85) dependent on the position of the first setting element relative to the second setting element (74 and 86 respectively), which control arrangement has an engagement element (85) that by the axial displacement of the cocking element (74) serving as the first setting element can be made selectively to engage with a first portion (110) of a counterpart element (62) after the cocking and with a second portion (113) thereof after the injection process,

which first portion (110) is provided with control recesses (100) for engagement with the engagement element (85), which upon engagement with the engagement element (85), in cooperation with it, enable a relative rotation between the engagement element (85) and these control recesses (100) only in a direction that increases the injection dose.

10. Injection device according to one or more of the preceding claims, characterised in that the second setting element (86) is rotatable relative to the cocking element (74) serving as the first setting element, and in that between them a scale indicator (87, 88, 89) is provided which indicates the relative rotational position between them and is preferably embodied as a indicator for the set injection dose.

11. Injection device according to one or more of the preceding claims, characterised in that the second setting element (86) is rotatably connected to the housing (32) of the injection device (10) via a slip coupling (79).

12. Injection device according to claim 11, characterised in that the slip coupling (79) is embodied in order to enable a rotation of the setting element (86) relative to the housing (32) only above a predetermined minimum torque acting upon the setting element.

13. Injection device according to one or more of the preceding claims, characterised in that the second setting element (86) at least partly surrounds the cocking element (74) in its cocked state - and preferably in its uncocked state as well - in order to prevent the fingers or the like of the user from being pinched between the second setting element (86) and the cocking element (74) serving as the first setting element.

14. Injection device according to one or more of the preceding claims, characterised in that scale markings (89) are provided on the circumference of the cocking element (74) serving as the first setting element, and that the second setting element (86) has a first recess (87), in which, in the cocked state of the cocking element (74), the indicated value of the scale marking (89) set by adjusting the cocking element is located.

15. Injection device according to one or more of the preceding claims, characterised in that scale markings (89) are provided on the circumference of the cocking element (74), and that the second setting element (86) comprises a second recess (88), in which, in the uncocked state of the cocking element (74), the indicated value of the scale marking (89) set by adjusting the cocking element is located.

16. Injection device according to one or more of the preceding claims, characterised in that the counterpart element (62) is embodied as a component of, or is connected to, the mechanism (18, 62) used to effect an injection process.

17. Injection device according to claim 16, characterised in that the mechanism (18, 62) which is adjustable in length has a threaded spindle (18), and in that the counterpart element (62) has a female thread (61) for guiding this threaded spindle (18).

18. Injection device according to claim 16 or 17, characterised in that the counterpart element (62) is provided on its end oriented toward the threaded spindle (18) with a guide element (66), preferably embodied as a plastic part, which is rotatably secured to the end (104) of the counterpart element (62) oriented toward the threaded spindle (18), engages (65) in a longitudinal groove (63) of the threaded spindle (18), and in turn is guided in the longitudinal direction (41) in the housing (30, 32) of the injection device (10), the guide element (66) being preferably snapped onto the associated end (104) of the counterpart element (62).

19. Injection device according to claim 18, characterised in that the guide element (66) is guided in the longitudinal direction by an extension (70, 71) thereof in the housing (30, 32) of the injection device (10), and in that this extension (70, 71) is embodied as a latch member which in the cocked position (Figs. 4, 5) engages in a corresponding detent recess (42) and thereby retains the mechanism (18, 62) used to effect the injection process in this cocked position (Figs. 4, 5).

20. Injection device according to claim 19, characterised in that the detent recess (42) is embodied as a recess in the housing (32) of the injection device (10), and in that a tripping mechanism actuatable from the outside, in particular a fastening clip (11), is provided for releasing the latch member (70, 71) and hence for initiating the injection process.

21. Injection device according to one or more of the preceding claims, characterised in that the container (12, 20) for the fluid to be injected is displaceable counter to the action of a restoring spring (29) up to a stop (46), in order, upon the release of the mechanism (18, 62) used to effect an injection process, to effect, firstly, a displacement of this container (12, 20) as far as this stop (46) and thereby a movement of the hypodermic needle (16) in the insertion direction, and subsequently an action upon the piston (17) in this container (12, 20) and thereby an injection process.

## Revendications

1. Appareil d'injection comportant un organe (18,62) réglable en longueur, servant à effectuer une opération d'injection, lequel organe peut être mis en position d'armement (figures 4, 5) par un coulissement longitudinal d'un organe d'armement (74) servant de premier organe de réglage, en agissant contre la force d'un ressort (105), et peut être libéré de cette position pour l'opération d'injection, et pour venir agir contre une paroi, en particulier un piston (17), d'un récipient (12) pour un liquide à injecter,
lequel organe peut être réglé en longueur pour obtenir le réglage d'une dose injectable souhaitée, par une rotation de l'organe d'armement (74), servant de premier organe de réglage,
comportant, en outre un deuxième organe de réglage (86) et un dispositif de commande (62, 85) dépendant de la position du premier organe de réglage par rapport au deuxième organe de réglage (respectivement 74 et 86), dispositif qui présente un organe d'enclenchement (85) qui, par le coulissement axial de l'organe d'armement (74) servant de premier organe de réglage, peut être enclenché sélectivement, après l'armement, avec une première partie (110), et, après l'opération d'injection, avec une deuxième partie (113) d'un organe conjugué (62),
cette dernière présentant une butée (117) pour l'enclenchement avec l'organe d'enclenchement (85), qui, si l'organe d'enclenchement (85) est enclenché avec la deuxième partie (113), limite, à une zone angulaire prédéterminée, la rotation relative entre l'organe d'armement (74) et le deuxième organe de réglage (86).

2. Appareil d'injection comportant un organe (18,62) réglable en longueur, servant à effectuer une opération d'injection, lequel organe peut être mis en position d'armement (figures 4, 5), par coulissement longitudinal d'un organe d'armement (74) servant de premier organe de réglage, en agissant contre la force d'un ressort (105), et peut être libéré de cette position pour l'opération d'injection, et pour venir agir contre une paroi, en particulier un piston (17), d'un récipient (12) pour un liquide à injecter,
lequel organe peut être réglé en longueur pour obtenir le réglage d'une dose injectable souhaitée, par une rotation de l'organe d'armement (74), servant de premier organe de réglage,
comportant en outre un deuxième organe de réglage (86) et avec un dispositif de commande (62, 85) dépendant de la position du premier organe de réglage par rapport au deuxième organe de réglage (respectivement 74 et 86), dispositif qui présente un organe d'enclenchement (85) qui, par le coulissement axial de l'organe d'armement (74) servant de premier organe de réglage, peut être enclenché sélectivement, après l'armement, avec une première partie (110), et, après l'opération d'injection, avec une deuxième partie (113) d'un organe conjugué (62),
cette dernière présentant un évidement de commande (116, 122) pour l'engagement avec l'organe d'enclenchement (85) qui, si l'organe d'enclenchement (85) est engagé, donc après l'opération d'injection, empêche un déplacement axial armant l'organe d'armement (74), en dehors d'une position de rotation relative prédéterminée entre l'organe d'enclenchement (85) et l'organe conjugué (62).

3. Appareil d'injection suivant la revendication 2, caractérisé en ce que la position de repos relative correspond à la valeur zéro d'une échelle graduée (89) prévue entre l'organe d'armement (74) et le deuxième organe de réglage (86).

4. Appareil d'injection suivant la revendication 3, caractérisé en ce que la deuxième partie de l'organe conjugué (62) est réalisée sous la forme d'une rainure annulaire (113), contre un bord de laquelle porte l'organe d'enclenchement (85), empêchant ainsi un mouvement d'armement axial de l'organe d'armement (74), quand cet organe d'armement (74) n'est pas armé, et en ce que, dans la position angulaire prédéterminée (figure 9), ou la plage angulaire prédéterminée, ce bord (116) présente un évidement (122) qui n'oppose pas d'obstacle à un déplacement axial d'armement de l'organe d'enclenchement (85).

5. Appareil d'injection comportant un organe (18,62) réglable en longueur, servant à effectuer une opération d'injection, lequel organe peut être mis en position d'armement (figures 4,5) par coulissement longitudinal d'un organe d'armement (74) servant de premier organe de réglage, en agissant contre la force d'un ressort (105), et peut être libéré de cette position pour effectuer une opération d'injection, et pour venir agir contre une paroi, en particulier un piston (17), d'un récipient (12) pour un liquide à injecter,
lequel organe peut être réglé en longueur pour obtenir le réglage d'une dose injectable souhaitée par une rota-

EP 0 362 222 B1

tion de l'organe d'armement (74), servant de premier organe de réglage,

comportant en outre un deuxième organe de réglage (86) et un dispositif de commande (62, 85) dépendant de la position du premier organe de réglage par rapport au deuxième organe de réglage (respectivement 74 et 86), ce dispositif présentant un organe d'enclenchement (85) qui, par le coulissement axial de l'organe d'armement (74) servant de premier organe de réglage, peut être enclenché sélectivement, après l'armement, avec une première partie (110), et, après l'opération d'injection, avec une deuxième partie (113) d'un organe conjugué (62),

cette première partie (110) étant équipée de dents de crantage (126) pour l'enclenchement de l'organe d'enclenchement (85), qui, si l'organe d'enclenchement (85) est enclenché, permettent une rotation pas-à-pas entre le premier et le deuxième organe de réglage (respectivement 74 et 85) et, ainsi, le réglage de la dose d'injection, en particulier au touche ou à l'oreille.

6. Appareil d'injection suivant la revendication 5, caractérisé en ce que la forme des dents de crantage (126) est réalisée, dans la direction de la deuxième partie (113) de l'organe conjugué (62), de telle façon que, lors du mouvement d'injection de l'organe (18, 62) servant à effectuer une opération d'injection, cette forme permette un glissement axial vers l'extérieur de l'organe d'enclenchement (85), en sortant des dents de crantage (126).

7. Appareil d'injection comportant un organe (18,62) réglable en longueur, servant à effectuer une opération d'injection, lequel organe peut être mis en position d'armement (figures 4, 5) par coulissement longitudinal d'un organe d'armement (74) servant de premier organe de réglage, en agissant contre la force d'un ressort (105), et peut être libéré de cette position pour effectuer une injection, pour venir agir contre une paroi, en particulier un piston (17), d'un récipient (12) pour un liquide à injecter,

lequel organe peut être réglé en longueur pour obtenir le réglage d'une dose injectable souhaitée, par une rotation de l'organe d'armement (74), servant de premier organe de réglage,

comportant en outre un deuxième organe de réglage (86) et un dispositif de commande (62, 85) dépendant de la position du premier organe de réglage par rapport au deuxième organe de réglage (respectivement 74 et 86), dispositif qui présente un organe d'enclenchement (85) qui, par le coulissement axial de l'organe d'armement (74) servant de premier organe de réglage, peut être enclenché sélectivement, après l'armement, avec une première partie (110), et, après l'opération d'injection, avec une deuxième partie (113) d'un organe conjugué (62),

cette première partie (110) présentant un dispositif de blocage (124) qui, pour une position relative de rotation prédéterminée entre l'organe d'enclenchement (85) et l'organe conjugué (62), bloque dans la position d'armement un mouvement d'injection de l'organe (18, 62) servant à effectuer une injection, par enclenchement du dispositif de blocage (124) avec l'organe d'enclenchement (85).

8. Appareil d'injection comportant un organe (18, 62) réglable en longueur, servant à effectuer une opération d'injection, lequel organe peut être mis en position d'armement (figures 4, 5), par coulissement longitudinal d'un organe d'armement (74) servant de premier organe de réglage, en agissant contre la force d'un ressort (105), et peut être libéré de cette position pour l'opération d'injection, pour venir agir contre une paroi, en particulier un piston (17), d'un récipient (12) pour un liquide à injecter,

lequel organe peut être réglé en longueur pour obtenir le réglage d'une dose injectable souhaitée, par une rotation de l'organe d'armement (74), servant de premier organe de réglage,

comportant, en outre un deuxième organe de réglage (86) et un dispositif de commande (62, 85) dépendant de la position du premier organe de réglage par rapport au deuxième organe de réglage (respectivement 74 et 86), dispositif qui présente un organe d'enclenchement (85) qui, par le coulissement axial de l'organe d'armement (74), servant de premier organe de réglage, peut être enclenché sélectivement, après l'armement, avec une première partie (110), et, après l'opération d'injection, avec une deuxième partie (113) d'un organe conjugué (62),

cette première partie (110) présentant une butée (136) pour l'enclenchement avec l'organe d'enclenchement (85), butée qui, quand l'organe d'enclenchement (85) est enclenché, limite la rotation entre l'organe d'enclenchement (85) et l'organe conjugué (62) à une zone angulaire de rotation prédéterminée, pour limiter vers le haut la dose réglable d'injection.

9. Appareil d'injection comportant un organe (18, 62) réglable en longueur, servant à effectuer une opération d'injection, lequel organe peut être mis en position d'armement (figures 4, 5), par coulissement longitudinal d'un organe d'armement (74) servant de premier organe de réglage, en agissant contre la force d'un ressort (105) et peut être libéré de cette position pour l'opération d'injection, pour venir agir contre une paroi, en particulier un piston (17), d'un récipient (12) pour un liquide à injecter,

lequel organe peut être réglé en longueur pour obtenir le réglage d'une dose injectable souhaitée, par une rotation de l'organe d'armement (74), servant de premier organe de réglage,

comportant en outre un deuxième organe de réglage (86) et un dispositif de commande (62, 85) dépendant

17

de la position du premier organe de réglage par rapport au deuxième organe de réglage (respectivement 74 et 86), dispositif qui présente un organe d'enclenchement (85) qui, par le coulissement axial de l'organe d'armement (74) servant de premier organe de réglage, peut être enclenché sélectivement, après l'armement, avec une première partie (110), et, après l'opération d'injection, avec une deuxième partie (113) d'un organe conjugué (62),

cette première partie (110) présentant des évidements de commande (100) pour l'enclenchement avec l'organe d'enclenchement (85), évidements qui, quand l'organe d'enclenchement (85) est enclenché, en coopération avec celui-ci, ne permettent un mouvement relatif de rotation entre l'organe d'enclenchement (85) et ces évidements de commande (100) que dans une direction augmentant la dose d'injection.

10. Appareil d'injection suivant l'une ou plusieurs des revendications précédentes, caractérisé en ce que le deuxième organe de réglage (86) peut être tourné par rapport à l'organe d'armement (74) servant de premier organe de réglage, et en ce qu'entre eux, est prévue une échelle graduée (87, 88, 89), indiquant leur position angulaire relative entre eux et, de préférence, réalisée sous la forme d'un indicateur de la dose d'injection qui à été réglée.

11. Appareil d'injection suivant l'une ou plusieurs des revendications précédentes, caractérisé en ce que le deuxième organe de réglage (86) est relié à rotation, par l'intermédiaire d'un accouplement à friction (79), au boîtier (32) de l'appareil d'injection (10).

12. Appareil d'injection suivant la revendication 11, caractérisé en ce que l'accouplement à friction (79) est réalisé de façon à ne permettre à l'organe de réglage (86) une rotation par rapport au boîtier (32) qu'au dessus d'un couple de rotation minimal prédéterminé agissant sur cet organe.

13. Appareil d'injection suivant l'une ou plusieurs des revendications précédentes, caractérisé en ce que le deuxième organe de réglage (86) entoure, au moins en partie, l'organe d'armement (74) dans sa position d'armement - et, de préférence, également dans sa position non armée - pour empêcher un coincement du doigt, ou un incident similaire, de l'utilisateur entre le deuxième organe de réglage (86) et l'organe d'armement (74) servant de premier organe de réglage.

14. Appareil d'injection suivant l'une ou plusieurs des revendications précédentes, caractérisé en ce que, sur la périphérie de l'organe d'armement (74), sont prévues des graduations d'une échelle (89), et en ce que le deuxième organe de réglage (86) présente un premier évidement (87) dans lequel se trouve, dans la position d'armement de l'organe de réglage (74), l'indication de la valeur de l'échelle graduée (89), telle que cette valeur a été réglée par le positionnement de l'organe d'armement.

15. Appareil d'injection suivant l'une ou plusieurs des revendications précédentes, caractérisé en ce que, sur la périphérie de l'organe d'armement (74), sont prévues des graduations d'une échelle (89), et en ce que le deuxième organe de réglage (86) présente un deuxième évidement (88) dans lequel se trouve, dans la position non armée de l'organe d'armement (74), la valeur indiquée de l'échelle graduée (89), telle que cette valeur a été réglée par le positionnement de l'organe d'armement.

16. Appareil d'injection suivant l'une ou plusieurs des revendications précédentes, caractérisé en ce que l'organe conjugué (62) est réalisé sous la forme d'une pièce constitutive de l'organe (18, 62) servant à effectuer une opération d'injection, ou est relié à celui-ci.

17. Appareil d'injection suivant la revendication 16, caractérisé en ce que l'organe (18, 62), réglable en longueur, présente une broche filetée (18) et en ce que l'organe conjugué (62) présente un filetage intérieur (61) pour le guidage de cette broche filetée (18).

18. Appareil d'injection suivant la revendication 16 ou la revendication 17, caractérisé en ce que l'organe conjugué (62) est équipé, à son extrémité tournée vers la broche filetée (18), d'un organe de guidage (66), réalisé, de préférence, sous la forme d'une pièce en matière plastique, cet organe de guidage étant fixé, de manière rotative, sur l'extrémité (104) de l'organe conjugué (62) qui est orientée vers la broche filetée (18), s'enclenche dans une rainure longitudinale (63) de la broche filetée (18) et, de son côté, est guidé dans le boîtier (30, 32) de l'appareil d'injection (10) suivant la direction longitudinale (41), l'organe de guidage (66) étant, de préférence, fixé par enclenchement sur l'extrémité correspondante (104) de l'organe conjugué (62).

19. Appareil d'injection suivant la revendication 18, caractérisé en ce que l'organe de guidage (66) est guidé, suivant la direction longitudinale, par un prolongement (70, 71) dans le boîtier (30, 32) de l'appareil d'injection (10),

et en ce que ce prolongement (70, 71) est réalisé pour former un organe d'arrêt qui s'enclenche, en position d'armement (figures 4, 5), dans un évidement d'arrêt (42) correspondant et, ainsi, maintient fixe, dans cette position d'armement (figures 4, 5), l'organe (18, 62) servant à effectuer l'opération d'injection.

20. Appareil d'injection suivant la revendication 19, caractérisé en ce que l'évidaient d'arrêt (42) est réalisé sous la forme d'un perçage dans le boîtier (32) de l'appareil d'injection (10) et en ce qu'il est prévu un organe de déclenchement, actionnable de l'extérieur, en particulier un clip de fixation (11), pour libérer l'organe d'arrêt (70, 71), donc pour déclencher l'opération d'injection.

21. Appareil d'injection suivant l'une ou plusieurs des revendications précédentes, caractérisé en ce que le récipient (12, 20) pour le liquide à injecter peut coulisser, en agissant contre un ressort de rappel (29), jusqu'à une butée (46), pour qu'en libérant l'organe (18, 62), servant à effectuer l'opération d'injection, on provoque d'abord un coulissement de ce récipient (12, 20) jusqu'à cette butée (46) et, ainsi, un déplacement de la canule d'injection (16) dans la direction de la piqûre et qu'ensuite on vienne agir contre le piston (17) dans ce récipient (12, 20) et, de ce fait, on effectue une opération d'injection.

Fig. 1

Fig.2

Fig.3

Fig.4

23

EP 0 362 222 B1

*Fig.5*

24

Fig. 6

EP 0 362 222 B1

Fig. 7

Fig.8

EP 0 362 222 B1

Fig. 9

Fig. 10

Fig. 11

Fig. 12

Fig. 13

Fig. 14

Fig. 14A

27

Fig. 15

*Fig. 16*

*Fig. 17*

*Fig. 18*

*Fig. 19*

*Fig. 20*

29

Fig. 21

Fig. 22

Fig. 23

Fig. 24

EP 0 362 222 B1

Fig. 25

Fig. 26

Fig. 27

Fig. 28